(19) Europäisches Patentamt / European Patent Office / Office européen des brevets

(11) **EP 2 595 606 B1**

(12) **EUROPEAN PATENT SPECIFICATION**

(45) Date of publication and mention
of the grant of the patent:
**09.05.2018 Bulletin 2018/19**

(51) Int Cl.:
*A61K 9/14* (2006.01)   *A61K 9/22* (2006.01)
*A61K 31/57* (2006.01)   *C08J 3/12* (2006.01)
*A61K 9/16* (2006.01)   *A61K 31/551* (2006.01)
*A61K 31/4196* (2006.01)   *C08J 3/05* (2006.01)
*C08J 3/16* (2006.01)

(21) Application number: **11809867.2**

(22) Date of filing: **20.07.2011**

(86) International application number:
**PCT/KR2011/005347**

(87) International publication number:
**WO 2012/011740 (26.01.2012 Gazette 2012/04)**

(54) **METHOD FOR PREPARING MICROSPHERES AND MICROSPHERES PRODUCED THEREBY**

VERFAHREN ZUR HERSTELLUNG VON MIKROKUGELN UND IN DIESEM VERFAHREN HERGESTELLTE MIKROKUGELN

PROCÉDÉ DE PRÉPARATION DE MICROSPHÈRES ET MICROSPHÈRES PRODUITES PAR CE PROCÉDÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.07.2010 KR 20100070407**

(43) Date of publication of application:
**29.05.2013 Bulletin 2013/22**

(73) Proprietors:
• **SK Chemicals Co., Ltd.**
**Seongnam-si, Gyeonggi-do 463-400 (KR)**
• **Ewha University-Industry Collaboration Foundation**
**120-750 Seoul (KR)**

(72) Inventors:
• **SAH, Hong Kee**
**Seoul 158-050 (KR)**
• **LEE, Bong-Yong**
**Seoul 137-935 (KR)**
• **UM, Key-An**
**Suwon-si**
**Gyeonggi-do 440-709 (KR)**
• **OH, Joon-gyo**
**Suwon-si**
**Gyeonggi-do 440-709 (KR)**
• **HWANG, Yong Youn**
**Suwon-si**
**Gyeonggi-do 440-806 (KR)**
• **KIM, Hong-Kee**
**Gunpo-si**
**Gyeonggi-do 435-040 (KR)**
• **LEE, Kyu Ho**
**Suwon-si**
**Gyeonggi-do 440-330 (KR)**
• **HONG, Seok Hyun**
**Seoul 137-845 (KR)**
• **LEE, Yoon-Jung**
**Yongin-si**
**Gyeonggi-do 446-951 (KR)**

(74) Representative: **Isarpatent**
**Patent- und Rechtsanwälte Behnisch Barth Charles**
**Hassa Peckmann & Partner mbB**
**Friedrichstrasse 31**
**80801 München (DE)**

(56) References cited:
**WO-A2-2010/024615     CA-A1- 2 743 600**
**JP-A- 2003 212 758     KR-A- 20020 093 097**
**US-A1- 2009 318 569     US-A1- 2009 318 569**
**US-B1- 6 403 114**

**(Cont. next page)**

- **Yogita Bahl ET AL: "Dynamic changes in size distribution of emulsion droplets during ethyl acetate-based microencapsulation process", AAPS PharmSciTech, 1 March 2000 (2000-03-01), pages 41-49, XP55188631, DOI: 10.1007/BF02830520 Retrieved from the Internet: URL:http://www.ncbi.nlm.nih.gov/pmc/articl es/PMC2784832/pdf/12249_2008_Article_1141. pdf [retrieved on 2015-05-11]**

## Description

[Technical Field]

The present invention relates to a method for preparing microsphere and microspheres produced by thereby. The present invention relates to a method for preparing a polymeric microsphere as defined in the claims.

[Background Art]

Conventional injectable formulations such as solution, suspension, and emulsion are quickly removed from the body after administration, and therefore frequent administration is essentially needed for treatment of chronic diseases. Microencapsulation has been developed to solve the problem, and referred to a production process for encapsulating drugs in microspheres (hereinafter, the term microsphere will include nanospheres) consisting of high molecular compounds. Microspheres are usually in a size of $\mu$m unit, and can be administered to a human or animal by intramuscular or subcutaneous injection. Further, microspheres can be produced to have a variety of drug release rates, so that the period of drug delivery can be controlled. Therefore, evenif a therapeutic drug is administered only once, its effective concentration can be maintained over a long period of time, and the total administration amount of therapeutic drug can be minimized to improve the drug compliance in patients. Accordingly, world famous pharmaceutical companies are very interested in the production of polymeric microsphere loaded with drugs.

In the production of polymeric microspheres by microencapsulation, poly-d,1-lactide-co-glycolide (PLGA) has been most widely used as a high molecular compound. PLGA is a biocompatible high molecular compound that is hydrolyzed in vivo to be converted into nontoxic lactic acid and glycolic acid. Therefore, pharmaceutical industries have made extensive studies on the development of drug formulation using PLGA, and examples of current available microsphere product produced by using PLGA include Risperdal Consta, Sandostatin LAR, Vivitrol, and Lupron Depot. Each of them is administered to a patient once to control the release of risperidone, octreotide acetate, naltrexone, and leuprolide acetate from 2 weeks to 4 months.

Such polymeric microspheres loaded with drugs can be conventionally produced by a solvent evaporation method or a solvent extraction method using an organic solvent such as methylene chloride and ethyl acetate.

First, the solvent evaporation method will be briefly described (see US PatentNos. 6,471,996, 5,985,309, and 5,271,945). A drug is dispersed or dissolved in an organic solvent in which a high molecular compound is dissolved, and then emulsified in a dispersion medium such as water to produce an oil-in-water(0/W) emulsion. Then the organic solvent in the emulsion is diffused into a dispersion medium and evaporated across the air/water interface to form the polymeric microspheres loaded with drugs. At this time, in order to accelerate the diffusion of organic solvent into the dispersion medium, a method such as organic solvent extraction using reduced pressure, increased temperature, and an excessive amount of water is used. A dispersion organic solvent that is generally used to dissolve the high molecular PLGA is methylene chloride, which dissolves a PLGA copolymer well using various molecular weights and lactide:glycolide ratios and because it does not mix well with water due to the low water solubility of 1.32% by weight. Thus, methylene chloride is a suitable solvent for the production of oil-in-water emulsion. Further, due to the low boiling point of 39.8°C, a small amount of methylene chloride molecules that diffused from emulsion liquid droplets into water are evaporated across the water/air interface. Such process is continuously repeated to remove methylene chloride from emulsion droplets, thereby forming microspheres. Finally, the residual methylene chloride present in microspheres is easily dried and removed due to its low boiling point.

Likewise, even though methylene chloride is the most optimal solvent used for the production of emulsion in that it is very volatile, not mixed well with water, and has a lower boiling point than water, methylene chloride has the following problems: (a) it is a carcinogen proved by experiments; (b) it destroys theozone layer in the atmosphere to generate a toxic environment, causing an increase in human skin cancer; (c) it is one of the 38 toxic and hazardous substances announced by the agency for toxic substances and disease registry within the US Department of Health and Human Services; (d) a lot of time is required to completely remove methylene chloride in the emulsion droplets,since it has a low water solubility of about 1.32% by weight and only a small amount of methylene chloride is dissolved in water and evaporates. For example, in US Pat No. 6,884,435, the emulsion is stirred overnight to remove methylene chloride from the emulsion, and conditions such as increased temperature or reduced pressure in a reactor are introduced to shorten the production time of microspheres (see US Pat Nos. 3,691,090, 3,891,570, 6,270,700, and 6,572,894).

On the other hand, the solvent extraction method used to produce polymeric microspheres loaded with drugs is a method for effectively extracting the organic solvent in the emulsion droplets by using a large amount of solubilizing solvent. When the organic solvent is extracted from the emulsion droplets, the dissolved high molecular compounds are hardened to convert the emulsion droplets into microspheres. The solubilizing solvent that is generally used is water, and the degree of water solubility of the organic solvent greatly affects the amount of water needed. For example, methylene chloride has water solubility of 1.32% by weight, whereby a very large amount of water is needed for extracting methylene chloride in the emulsion. However, a large amount of wastewater containing methylene chloride is produced, in which the treatment of the wastewater becomes a problematic issue. Therefore, in the solvent extraction method, ethyl acetate, which has higher water solubility than methylene chloride, is mainly used. Since ethyl acetate has the water solubility of 8.7% by weight, it can be extracted by using a relatively small amount of water, as compared to methylene chloride, and it is advantageously a nonhalogenated organic solvent. However, its boiling point is 77°C and much higher than 39.8°C, which is that of methylene chloride. Thus, ethyl acetate has a drawback that the residual solvent is hard to remove when dried. Furthermore, a high molecular PLGA compound with a specific molecular weight and lactide:glycolide ratio has a characteristic of not dissolving easily in ethyl acetate.

Therefore, technologies simultaneously employing the solvent evaporation method and solvent extraction method are disclosed in US Pat Nos. 4,389,840, 4,530,840, 6,544,559, 6,368,632, and 6,572,894. That is, in the methods, the emulsion is produced, and then the organic solvent is partially removed by the evaporation process, and the residual organic solvent is removed by the solvent extraction method. For example, US Pat No. 4,389,840 discloses a method for producing microspheres, in which a drug and a high molecular PLGA are dissolved in methylene chloride and then emulsified in water to produce oil in water-type emulsion, then 40 to 60% by weight of methylene chloride is removed by the evaporation process, and the residual methylene chloride is extracted using a large amount of water to produce microspheres.

US Pat. No. 6,403,114 discloses the use of an ethanol-containing solution in the washing step in order to diminish the residual solvent content.

However, since all of the organic solvents used in the known methods do not have sufficient high water solubility, excessively large amounts of water (over 10 times more than water solubility of organic solvent) should be used. Thus, a large-volume reactor is needed, and a large amount of wastewater containing organic solvent is produced, as a result, the cost for wastewater treatment is increased. Further, there is a problem that the residual organic solvent present in the microspheres is not effectively removed.

The present inventors disclosed an method for preparing microspheres containing a drug comprising the step of changing insoluble organic solvent to soluble organic solvent by adding ammonium solution in Korean Patent No. 10-0918092. Through the method, a microshperes could be prepared easily and quickly with minimizing generation of waste water. However, this method has still a problem that the amount of remaining organic solvent is over 1%.

In particular, when a large amount of organic solvent remains in the microspheres, the microspheres tend to coalesce during the drying process. As a result, since the microspheres may not be dispersed separately after the drying process, a problem may occur during injection and the reproducibility of drug release may decrease. Further, if the amount of the remaining solvent exceeds an allowable limit, it will be difficult to get the regulatory approval.

Therefore, the development of novel method minizing organic solvent remains in microspheres is urgent.

[Disclosure]

[Technical Problem]

Accordingly, the inventors of the present invention have conducted research on a method for reducing the amount of organic solvent remaining in a prepared polymeric microsphere, in a polymeric microsphere preparation method including a removal step of a water-insoluble organic solvent by an acid or base. As a result, they found that when a water-insoluble organic solvent used in dispersed phase preparation is previously added to a dispersion solvent, the concentration of organic solvent remaining in the prepared polymeric microsphere is further reduced. Then, they completed the present invention based on the findings.

Accordingly, an object of the present invention is to provide a method for preparing a polymeric microsphere.

A further object of the present invention is to provide a polymeric microsphere prepared by the above method.

A still further object of the present invention is to provide a drug delivery composition including, as an active ingredient, a polymeric microsphere prepared by the above method.

[Technical Solution]

The present invention provides a method for preparing a polymeric microsphere as defined in the claims.

In accordance with a further aspect of the present invention, there is provided a polymeric microsphere prepared by the above method.

In accordance with a still further object of the present invention, there is provided a drug delivery composition including, as an active ingredient, a polymeric microsphere prepared by the above method.

Hereinafter, the present invention will be described in more detail.

The method for preparing a polymeric microsphere, according to the present invention, includes the steps of:

a) mixing a water-insoluble organic solvent with a dispersion solvent;

b) mixing a polymer compound, a drug and a water-insoluble organic solvent so as to prepare a dispersed phase;

c) mixing the dispersed phase of step (b) with the dispersion solvent mixed with the water-insoluble organic solvent of step (a) so as to prepare an O/W (oil-in-water), O/O (oil-in-oil) or W/O/W (water-in oil-in-water) type emulsion;

d) adding a base or an acid to the emulsion of step (c) so as to remove the water-insoluble organic solvent from the emulsion and

(e) obtaining the polymeric microsphere prepared of step (d) and re-dispersing the obtained polymeric microsphere in a warmed dispersion solvent,

wherein the water-insoluble organic solvent in the steps (a) and (b) is selected from the group consisting of methyl acetate, ethyl acetate, propyl acetate, isopropyl acetate, butyl acetate, methyl formate, ethyl formate, isopropyl formate, propyl formate, butyl formate, methyl dichloroacetate, methyl chloroacetate, ethyl chloroacetate, ethyl dichloroacetate, methyl fluoroacetate, methyl difluoroacetate, ethyl fluoroacetate, ethyl difluoroacetate, maleic anhydride, acetic anhydride, propionic anhydride, phosphoric anhydride, acetamide, propionamide, and butylamide,

wherein the dispersion solvent is polyvinyl alcohol solution, or aqueous solution of polysorbates or a co-solvent thereof, or a non-aqueous dispersion solvent which is selected from the group consisting of silicone oil containing glycerin esters of fatty acids or lecithin, vegetable oil, toluene, and xylene,

wherein the base is selected from the group consisting of sodium hydroxide (NaOH), lithium hydroxide (LiOH), potassium hydroxide (KOH), ammonium hydroxide (NH 4 OH), copper hydroxide (Cu(OH)2), and iron hydroxide (Fe(OH)3),

wherein, in the step (c), a ratio of the dispersed phase of step (b) and the dispersion solvent of step (a) which is mixed with water-insoluble organic solvent is 1:4 to 20, and

wherein the temperature of the warmed dispersion solvent in the step (e) is 30°C to 50°C.

Hereinafter, a method for preparing a polymeric microsphere, according to the present invention, will be described in detail in respective steps.

In the step (a), a water-insoluble organic solvent and a dispersion solvent are mixed.

The water-insoluble organic solvent used in the present invention may be used without limitation if it is well known in the art, as long as it is capable of dissolving the polymer which is used for the preparation of the polymeric microspheres, being hydrolyzed by an acid or a base, and being hydrolyzed into water-soluble products. In general, compounds having backbone of amide, ester, anhydride and halogen acid are known to be easily hydrolyzed by an acid or a base. Compounds having backbone of anhydride are hydrolyzed to produce water-soluble carboxylic acids, and compounds having backbone of ester are hydrolyzed into carboxylic acid and alcohol. Compounds having backbone of acid halogen are hydrolyzed into carboxylic acid and halogen acid(HF, HCl, Hbr, HI etc). Since compounds having backbone of amide are hydrolyzed into carboxylic acid and amine, if the produced amine is water-soluble, the corresponding amide may be included in the water-insoluble organic solvent of the present invention.

The water-insoluble organic solvent of the present invention may be compounds having backbone of acid halogen, compounds having backbone of anhydride, compounds having backbone of phosphoric anhydride, compounds having backbone of ester, compounds having backbone of carboxylic esters, compounds having backbone of phosphoric esters, compounds having backbone of sulfuric acid esters, compounds having backbone of nitric esters, compounds having backbone of boric acid, compounds having backbone of amide and compounds having backbone of carboxylic amides, preferably, methyl acetate, ethyl acetate, propyl acetate, isopropyl acetate, butyl acetate, methyl formate, ethyl formate, isopropyl formate, propyl formate, butyl formate, methyl dichloroacetate, methyl chloroacetate, ethyl chloroacetate, ethyl dichloroacetate, methyl fluoroacetate, methyl difluoroacetate, ethyl fluoroacetate, ethyl difluoroacetate, maleic anhydride, acetic anhydride, propionic anhydride, phosphoric anhydride, acetamide, propionamide, butyl amide and carboxyl amide.

More specifically, it is preferable that the water-insoluble organic solvent is selected from the group consisting of ethyl acetate, methyl acetate, methyl formate, ethyl formate, isopropyl formate, propyl formate, acetic anhydride or propionic anhydride.

The dispersion solvent used in the present invention includes an aqueous dispersion solvent containing an emulsifier or non-aqueous dispersion solvent, and the aqueous dispersion solvent is used for the preparation of an O/W type and W/O/W type emulsion, the non-aqueous dispersion solvent is used for the preparation of an O/O type emulsion. As the aqueous dispersion solvent, an aqueous solution containing hydrophilic emulsifier such as polyvinyl alcohol or polysorbates (for example, polysorbate 20, polysorbate 60, polysorbate 65, polysobate 80, polysorbate 85) or a co-solvent thereof can be used. As the non-aqueous dispersion solvent, silicone oil, vegetable oil, toluene, or xylene containing lipophilic emulsifier such as glycerin esters of fatty acids or lecithin can be used. The concentration of the emulsifier in the dispersion solvent may be 0.05 to 15% (w/v).

The amount of the water-insoluble organic solvent to be mixed with the dispersion solvent may vary according to the kind of the polymer compound used in the preparation of the polymeric microsphere, the kind of the drug to be loaded, and the kind of the dispersion solvent. Preferably, the water-insoluble organic solvent may be added in an amount less than its water solubility. When it is mixed in an excessively small amount, a polymeric microsphere has a porous surface structure, thereby increasing an initially released amount of a drug. Meanwhile, when it is added in an amount more than its water solubility, it is difficult to remove the organic solvent, thereby increasing the concentration of the remaining organic solvent.

In the step (b), a polymer compound, a drug and a water-insoluble organic solvent are mixed so as to prepare a dispersed phase.

In the present invention, "a dispersed phase" indicates that a polymer compound and a drug are dissolved in and mixed with a water-insoluble organic solvent.

The example of the water-insoluble organic solvent in the step (b) is the same as that described in the step (a).

In the step (b), the water-insoluble organic solvent may be preferably the same kind of organic solvent as that used in the step (a). As required, as the water-insoluble organic solvent, a cosolvent obtained by mixing the water-insoluble organic solvent with at least one kind of other organic solvent may be used so that the solubility of a drug to be loaded in the microsphere can be adjusted or the hardening rate of emulsion drops can be controlled as desired.

There is no limitation in the polymer compound used in the preparation method of the present invention, as long as it is a polymer compound known in the art. Preferably, the polymer compound may be selected from the group including polylactic acid, polylactide, polylactic-co-glycolic acid, polylactide-co-glycolide (PLGA), polyphosphazene, polyiminocarbonate, polyphosphoester, polyanhydride, polyorthoester, lactic acid-caprolactone copolymer, polycaprolactone, polyhydroxyvalerate, polyhydroxybutyrate, polyamino acid, lactic acid-amino acid copolymer, and a mixture thereof. More preferably, the polymer compound may be polylactide-co-glycolide (PLGA).

Also, preferably, the polymer compound used in the preparation method of the present invention may be a polymer compound whose end is treated so that it is not subjected to hydrolysis by acid or base. For example, the polymer compound may be PLGA with an esterified end, PCL with an esterified end, or polyanhydride with an esterified end.

The drug used in the present invention may include all of hydrophilic drugs and hydrophobic drugs and it may be used without limitation if it is able to be encapsulated to polymeric microshperes. Examples of the drug include progesterone, haloperidol, thiothixene, olanzapine, clozapine, bromperidol, pimozide, risperidone, ziprasidone, diazepma, ethyl loflazepate, alprazolam, nemonapride, fluoxetine, sertraline, venlafaxine, donepezil, tacrine, galantamine, rivastigmine, selegiline, ropinirole, pergolide, trihexyphenidyl, bromocriptine, benztropine, colchicine, nordazepam, etizolam, bromazepam, clotiazepam, mexazolum, buspirone, goserelin acetate, somatotropin, leuprolide acetate, octreotide, cetrorelix, sandostatin acetate, gonadotropin, fluconazole, itraconazole, mizoribine, cyclosporin, tacrolimus, naloxone, naltrexone, cladribine, chlorambucil, tretinoin, carmusitne, anagrelide, doxorubicin, anastrozole, idarubicin, cisplatin, dactinomycin, docetaxel, paclitaxel, raltitrexed, epirubicin, letrozole, mefloquine, primaquine, oxybutynin, tolterodine, allylestrenol, lovostatin, simvastatin, provastatin, atrovastatin, alendronate, salcatonin, raloxifene, oxadrolone, conjugated estrogen, estradiol, estradiol valerate, estradiol benzoate, ethinyl estradiol, etonogestrel, levonorgestrel, tibolone, norethisterone and piroxicam and it also may be macro molecules such as proteins or nucleic acid.

The polymer compound may be used in an amount of 1 to 500 parts by weight, preferably of 1 to 50 parts by weight, with respect to 1 part by weight of a drug.

In the step (c), the dispersed phase in the step (b) is mixed with the dispersion solvent mixed with the water-insoluble organic solvent in the step (a) so as to prepare an O/W (oil-in-water), O/O (oil-in-oil) or W/O/W (water-in oil-in-water) type emulsion.

In the preparation of the O/W (oil-in-water) type emulsion, a polymer compound, a drug and a water-insoluble organic solvent are mixed to prepare a dispersed phase, and the dispersed phase is mixed with a dispersion solvent added with a water-insoluble organic solvent to prepare the O/W (oil-in-water) type emulsion. In the preparation of the O/O (oil in oil) type emulsion, a polymer compound, a drug and an organic solvent are mixed to prepare a dispersed phase, and the dispersed phase is mixed with a dispersion solvent including an organic solvent not to be mixed with the previously used organic solvent to prepare the O/O (oil in oil) type emulsion. In the preparation of the W/O/W (water-in-oil-in-water) type emulsion, an aqueous solution having a drug dissolved therein is emulsified in a water-insoluble organic solvent having a polymer compound dissolved therein to prepare a W/O (water-in-oil) type emulsion, and the prepared emulsion is mixed again with a dispersion solvent added with a water-insoluble organic solvent to prepare a W/O/W (water-in-oil-in-water) type emulsion.

In the step (c), the dispersed phase and the dispersion solvent mixed with the water-insoluble organic solvent are mixed in a volumetric ratio of 1: 4 to 20.

When the ratio of the dispersion solvent is less than the above mentioned range, emulsion formation is not sufficiently carried out, and when the ratio is greater than the range, a waste solution excessively increased.

In the step (d), the emulsion prepared in the step (c) is added with a base or an acid so as to remove the water-insoluble organic solvent from the emulsion.

In the present invention, the step of adding a base or acid solution so as to remove the water-insoluble organic solvent from the emulsion is preferably carried out by a hydrolysis reaction. The hydrolysis reaction is a reaction where a corresponding material is split into two materials through the addition of water. In the hydrolysis reaction, a compound having an ester structure is hydrolyzed into carboxylic acid and alcohol, a compound having an anhydride structure is hydrolyzed into carboxylic acid, a compound having an amide structure is hydrolyzed into carboxylic acid and amine, and a compound having a halogen acid structure is hydrolyzed into carboxylic acid and halogen acid (HF, HCl, HBr, HI or the like). Through the reaction, the water-insoluble organic solvent diffused (or dissolved) in a small amount in one layer (e.g., a water phase) can be converted into a water soluble organic solvent capable of being completely dissolved in water, and by the converted amount, the water-insoluble organic solvent can be diffused into the water phase. While this process is continuously carried out, the water-insoluble organic solvent is removed from the inside of the emulsion, thereby hardening emulsion drops into microspheres. Thus, it is possible to prepare required drug-containing polymeric microspheres. The above described removal of the water-insoluble organic solvent from the inside of the emulsion indicates not only that the water-insoluble organic solvent is completely or actually (up to an extent where it is not detected) removed, but also that the amount of the water-insoluble organic solvent is reduced compared to that in the initial stage before the introduction of acid or base. Herein, since the emulsion drops are quickly hardened, interaction between emulsion drop particles is inhibited. This allows a required polymeric microsphere to be obtained without agglomeration. Herein, the acid catalyzes the reaction and the base is consumed by the reaction. Once the base or the acid is added, there is no particular problem in the reaction although the amount is less or greater than that of the water-insoluble organic solvent. However, addition of too many moles of acid or base may cause a problem in the stability of the drug and the polymer compound. Thus, it is required to consider a proper amount.

A basic solution may be added in such a manner that a molar ratio of the water-insoluble organic solvent to the basic solution can be preferably in a range of 1:0.1 to 10, more preferably of 1:0.2 to 5, much more preferably of 1:0.3 to 3, and most preferably of 1:0.5 to 1.5.

The temperature of the emulsion in the steps (c) and (d) may vary according to the kinds of the polymer compound, the drug, the water-insoluble organic solvent, and the base or acid, and preferably may be in a range of 0 °C to 35°C.

When the temperature of the emulsion is greater than 35°C, the drug and the polymer compound may be decomposed according to the kinds of the drug, the polymer compound, the base or acid. When the temperature is less than 0 °C, the water soluble dispersion solvent is frozen. Thus, emulsion formation cannot be smoothly carried out.

The base may be preferably selected from the group including sodium hydroxide (NaOH), lithium hydroxide (LiOH), potassium hydroxide (KOH), ammonium hydroxide ($NH_4OH$), copper hydroxide ($Cu(OH)_2$), and iron hydroxide ($Fe(OH)_3$), and the acid may be preferably selected from the group including hydrochloric acid (HCl), nitric acid ($HNO_3$), sulfuric acid ($H_2SO_4$), acetic acid ($CH_3COOH$), boric acid ($H_3BO_3$) and carbonic acid ($H_2CO_3$).

As described above, according to the present invention, it is possible to conveniently prepare a drug-containing polymeric microsphere within a short time by using a small amount of water, with the generation of a minimized amount of waste solution without a requirement of a conventional solvent-evaporation or solvent-extraction process. Also, according to the method for preparing a polymeric microsphere, according to the present invention, it is possible to prepare a polymeric microsphere with a low concentration of the organic solvent remaining within the polymeric microsphere.

In step (e), the polymeric microsphere prepared in the step (d), whose water-insoluble organic solvent is removed, is obtained, and then re-dispersed in a warmed dispersion solvent.

The dispersion solvent used in the re-dispersion of the present invention includes an aqueous dispersion solvent or a non-aqueous dispersion solvent which contains an emulsifying agent. In the preparation of an O/W or W/O/W type emulsion, the aqueous dispersion solvent may be used, and in the preparation of an O/O type emulsion, the non-aqueous re-dispersion solvent may be used. As the aqueous dispersion solvent, an aqueous solution or a cosolvent thereof may be used, which contains a hydrophilic emulsifier, such as polyvinyl alcohol and Polysorbate-based (e.g., polysorbate 20, polysorbate 60, polysorbate 65, polysorbate 80, polysorbate 85) emulsifier. As the non-aqueous dispersion solvent, silicon oil, vegetable oil, toluene, or xylene may be used, which contain a hydrophobic emulsifier, such as Glycerin Esters of Fatty Acids, or lecithin. The concentration of the emulsifier contained in the dispersion solvent may be in a range of 0.05 to 15% (w/v).

The temperature of the warmed dispersion solvent may vary according to the kinds and amounts of the drug, the water-insoluble organic solvent, and the polymer compound, and is in the range of 30°C to 50°C. When the temperature of the dispersion solvent is less than 20°C, the amount of remaining organic solvent may be increased. Then the temperature is greater than 80°C, the polymeric microsphere may be deformed.

In the polymeric microsphere re-dispersed in the warmed dispersion solvent, the concentration of the organic solvent within the microsphere is further reduced.

As described above, in the inventive method, it is possible to conveniently prepare a drug-containing polymeric microsphere within a short time by using a small amount of water, with the generation of a minimized amount of waste solution without a requirement of a conventional solvent-evaporation or solvent-extraction process. Also, it is possible to reduce the concentration of the organic solvent remaining within the prepared polymeric microsphere.

The polymeric microsphere prepared by the inventive method has a low concentration of remaining organic solvent, in which the releasing speed of a drug within a body can be controlled.

he polymeric microsphere prepared by the inventive method has an average diameter in a range of 0.1 to 1000$\mu$m, and preferably of 10 to 100$\mu$m, and may contain a drug in various weights as required.

Also, when the inventive polymeric microsphere is used, it is possible to effectively deliver the drug contained in the polymeric microsphere. Thus, the present invention provides a drug delivery composition including, as an active ingredient, the polymeric microsphere prepared by the inventive preparation method.

The inventive drug delivery composition has different target diseases according to its contained drugs, which may be easily understood by a person skilled in the art.

In Example 1 (not in the scope of the claims), a polymeric microsphere was prepared while the concentration of a water-insoluble organic solvent mixed with a dispersion solvent is varied. Then, according to such a concentration change, the concentration of remaining solvent was measured. As a result, it was found that as long as the dispersion solvent is previously added with the organic solvent, the concentration of the remaining organic solvent is reduced up to a maximum of about 25%, irrespective of drug inclusion.

In Example 2-1 (not in the scope of the claims), concentrations of a remaining solvent according to the temperature of a dispersion solvent in a re-dispersion step were compared. A polymeric microsphere preparation process was divided into two steps. In the first step, a base or acid solution is added and the reaction is completed, and in the second step, after the completion of the reaction, a microsphere is filtered and separated, and then is re-dispersed in PVA solution, followed by stirring. In the preparation of a polymeric microsphere containing 60mg of olanzapine, one group of polymeric microspheres was prepared while the temperatures of the first and second steps were warmed and maintained up to 33°C. On the other hand, the other group of polymeric microspheres was prepared while the temperature of only one of the first and second steps was warmed and maintained up to 33°C. Then, the remaining solvents were measured. As a result, it was found that irrespective of the warmed temperature in the first step, the warmed temperature in the second step significantly reduced the concentration of the solvent remaining in the polymeric microsphere, compared to the case where in the second step, the temperature is not warmed.

In Example 2-2 (not in the scope of the claims), the same result as the above described result was obtained when another kind of PLGA was used to repeatedly carry out the test.

In Example 2-3 (not in the scope of the claims), a polymeric microsphere was prepared by increasing the content of a polymer and the amount of a drug, and the amount of remaining solvent was measured. As a result, it was found that when the temperature in the re-dispersion step (second step) is warmed, it is possible to maintain a low amount of the remaining solvent.

In Example 3 (not in the scope of the claims), concentrations of a remaining solvent according to the concentrations of a water-insoluble organic solvent mixed with a dispersion solvent were compared. A polymer compound was dissolved in ethyl formate so as to provide a dispersed phase. The dispersed phase was emulsified in 20ml of 0.5% PVA aqueous solution so as to provide an emulsion, in which the PVA aqueous solution was previously added with 0, 1, 2 or 4ml of ethyl formate. Then, base was added to remove the water-insoluble organic solvent, and then the polymeric microsphere was prepared. Then, the amount of the remaining solvent was measured, and the polymeric microsphere was photographed.

As a result, it was found that as the concentration of an organic solvent previously added to a dispersion solvent is increased, the concentration of the solvent remaining in the polymeric microsphere is decreased. Furthermore, it was found that when an organic solvent is previously added to a dispersion solvent, it is possible to stably prepare a spherical polymeric microsphere.

In Example 4 (not in the scope of the claims), without a step of previously mixing a water-insoluble organic solvent with a dispersion solvent, polymeric microspheres were prepared by varying a ratio of a dispersed phase to the dispersion solvent. Then, according to a change of a ratio of a dispersed phase to a dispersion solvent, a change of the concentration of a remaining solvent was measured. A polymeric microsphere was prepared while the ratio of the dispersed phase to the dispersion solvent was adjusted to 1:10, 1:6 or 1:4. Then, the amount of remaining solvent was measured.

As a result, when the ratio of the dispersed phase and the dispersion solvent is 1:4 or 1:6, the amount of a remaining solvent (EA or EF) is reduced, compared to that in 1:10. However, it was found that the amount of remaining ethanol is increased. Thus, it was determined that it is possible to adjust the concentration of solvent remaining in the polymeric microsphere by adjusting the ratio of the dispersed phase to the dispersion solvent. Further, it was found that it doesn' t matter which one of the dispersed phase and the dispersion solvent in the ratio of the dispersed phase and the dispersion solvent was adjusted.

In Example 5-1, by varying the ratio of a dispersed phase to a dispersion solvent, and the amount of an organic solvent previously mixed with the dispersion solvent, and maintaining the temperature in the re-dispersion step at 40°C, polymeric microspheres were prepared. The change in the characteristics was measured. The dispersed phase was immobilized by 4ml of ethyl formate including 250mg of polymer compound dissolved therein. Then, the dispersion solvent was used in an amount of 20, 30, or 40ml, and the amount of the organic solvent previously added to the dispersion solvent was increased from 0% to 10% with respect to the dispersion solvent so as to provide a polymeric microsphere

As a result, when the amount of the remaining solvent was measured, compared to the case where no ethyl acetate was added to the dispersion solvent, the addition of ethyl acetate reduced the concentration of the remaining solvent. Especially, the amount of the remaining solvent was highly increased when a ratio of the dispersed phase to the dispersion solvent was 1:10 (40ml of dispersion solvent PVA) and the dispersion solvent was not previously added with EF.

In Example 5-2, the yield of the prepared polymeric microsphere was measured. As a result, it was determined that there is no problem in the yield of the polymeric microsphere.

In Example 5-3, a molecular weight change of a polymer compound used in the polymeric microsphere preparation, before/after the preparation, was measured. As a result, it was found that there was no significant difference in the molecular weights between the polymer compounds before/after the preparation.

In Example 6, a molecular weight change of a polymer compound before/after the preparation of a drug-containing polymeric microsphere was measured. The polymeric microsphere was prepared by varying the kind of a polymer compound, the kind and the content of a drug, and the temperature in first and second steps. Then, the molecular weight of the polymer compound before/after the preparation was measured.

As a result, it was found that polymer compounds of all the polymeric microspheres show no change in their molecular weights. Thus, it was determined that in the inventive preparation method, a molecular weight of a polymer compound can be well maintained irrespective of conditions such as the kind of a drug, or the reaction temperature.

In Example 7-2, an olanzapine-containing polymeric microsphere was prepared according to the inventive method.

For the prepared olanzapine-containing polymeric microsphere, the amount of remaining solvent, the loading efficiency of olanzapine, and the yield of the polymeric microsphere were measured. As a result, it was found that the low concentration of the remaining solvent was maintained, and almost all the loading efficiencies and the yields showed a high value of 70% or more.

In Example 8-2, a polymeric microsphere was prepared by using another water-insoluble organic solvent rather than the water-insoluble organic solvent used in Examples 1 to 7. By using ethyl acetate as the water-insoluble organic solvent, the polymeric microsphere was prepared. Then, the amount of remaining solvent, the loading efficiency of olanzapine, and the yield of the polymeric microsphere were measured. As a result, it was found that the small amount of the remaining solvent was maintained, and almost all the loading efficiencies and the yields showed high values of 85% or more and 70% or more, respectively.

In Example 9, an olanzapine-containing polymeric microsphere was prepared, and its dispersibility and injectability were tested. Then, it was injected into a rat. By measuring the concentration of olanzapine in blood, a pharmacokinetic test was carried out.

As a result, it was determined that the inventive composition including an olanzapine-containing polymeric microsphere is good in dispersibility and injectability, and thus can be used as a parenteral injection. Furthermore, it was found that when the composition was injected into a rat, olanzapine in blood was continuously released for a maximum of 80 days.

In Example 10-2, a polymeric microsphere containing anastrozole instead of olanzapine was prepared.

The yield and the loading efficiency of the prepared polymeric microsphere were almost 80% or more, which is appropriate irrespective of the kind of PLGA. Also, the average particle size was found to have an appropriate value ranging from 30 to 60um. The remaining solvent was included at a very low concentration, which was found to be appropriate irrespective of the kind of a test sample. Also, through the photograph by the electron microscope, it can be found that the polymeric microsphere was generally well prepared in a smooth spherical shape irrespective of the kind of used PLGA. Especially, it was found that even when the drug content was increased up to 40%, the drug was well loaded and no crystallization of the drug was observed from the surface.

In Example 10-3, through GPC analysis, the molecular weight change of a prepared polymeric microsphere was measured. As a result, it was found that most polymers show no change in their molecular weights. In order to analyze a related compound within the prepared polymeric microsphere, the prepared anastrozole-containing polymeric microsphere was subjected to ultra performance liquid chromatography (UPLC). As a result, it was found that the average amount of the related compound in the inventive polymeric microsphere was very low (0.17%).

Also, when a stability test was carried out for 2 months under a severe condition, the content of the related compound was less than 0.23%, and a change of the drug content was less than 10%. Thus, it was found that the polymeric microsphere prepared by the inventive preparation method has a high stability.

In Example 10-4, the persistence of a prepared polymeric microsphere was tested by introducing the inventive polymeric microsphere in a dialysis membrane, and changing a buffer at a predetermined time interval.

As a result, it was determined that after the drug release, polymeric microspheres of all formulations hardly have remaining drugs. Also, according to the kinds of the polymer compounds used in the preparation, as the ratio of lactide was increased, the drug was slowly released. Meanwhile, it was determined that as drug content was increased, the release rate was increased. Thus, it was determined that it is possible to adjust the release rate by adjusting the kind of the polymer compound used in the polymeric microsphere preparation, and the amount of the loaded drug.

In Example 10-5, the persistence of a prepared polymeric microsphere was measured through an animal test.

The prepared anastrozole-containing polymeric microsphere was administered in an amount of 20mg/kg to a rat through intramuscular injection, and then the concentration of anastrozole in blood was measured.

As a result, it was found that when the polymeric microsphere prepared by the inventive method was injected, a drug can be continuously released for a maximum of 120 days according to the kind of the prepared polymeric microsphere.

[Advantageous Effects]

As described above, the present invention provides a novel method for preparing a polymeric microsphere, the polymeric microsphere prepared by the method, and a drug delivery composition containing the polymeric microsphere, in which the method includes the steps of: mixing a water-insoluble organic solvent with a dispersion solvent; and removing the water-insoluble organic solvent from an emulsion by base or acid. In the inventive preparation method, it is possible to conveniently prepare a target drug-containing polymeric microsphere within a short time by using a small amount of water, with the generation of a minimized amount of waste solution without a requirement of a conventional solvent-evaporation or solvent-extraction process. Also, since a low concentration of remaining solvent within a prepared polymeric microsphere can be maintained, it is effective in preparation in sustained-release type medicines.

[Description of Drawings]

FIG. 1 shows a graph showing the measurement result of a remaining solvent of a polymeric microsphere prepared without previous addition of a water-insoluble organic solvent to a dispersion solvent;

FIG. 2 shows an electron microscopic photograph of an olanzapine-containing polymeric microsphere, in which FIG. 2A shows a polymeric microsphere prepared by maintaining the temperature in a preparation process at 33°C, and FIG. 2B shows a polymeric microsphere prepared without a warming step, in which in both cases, it can be found that the polymeric microsphere was well prepared in a spherical shape, and olanzapine was well loaded into the polymeric microsphere;

FIG. 3 shows an electron microscopic photograph of a polymeric microsphere prepared by maintaining the temperature in the second step at 40 °C, and increasing the amounts of a drug and a polymer compound, in which FIG. 3A shows a polymeric microsphere in a case where the amount of a drug is 60mg, and FIG. 3B shows a polymeric microsphere in a case where the amount of a drug is 80mg;

FIG. 4 shows a graph showing the concentration of a remaining solvent within a polymeric microsphere according to the concentration of an organic solvent previously added to a dispersion solvent;

FIG. 5 shows an electron microscopic photograph of a polymeric microsphere prepared by varying the concentration of an organic solvent previously added to a dispersion solvent (Add: amount (ml) of a water-insoluble organic solvent (ethyl formate) previously added to a dispersion solvent);

FIG. 6 shows a standard curve for measuring the molecular weight of a polymer compound before/after the polymeric microsphere preparation;

FIG. 7 shows an electron microscopic photograph of an olanzapine-containing polymeric microsphere (preparation no. is for an olanzapine-containing polymeric microsphere in Example);

FIG. 8 shows a graph showing the measurement result of a concentration change of olanzapine in the blood when a composition containing an olanzapine-containing polymeric microsphere was injected into a rat (preparation no. is for an olanzapine-containing polymeric microsphere in Example 9);

FIG. 9 shows an electron microscopic photograph of an anastrozole-containing polymeric microsphere (the number in the photograph indicates the preparation no. in Example 10, and the anastrozole-containing polymeric microsphere was prepared according to the composition of the corresponding preparation number);

FIG. 10 shows a graph showing the result of in vitro release test of an anastrozole-containing polymeric microsphere prepared by the inventive method; and

FIG. 11 shows a graph showing the result of an animal test on the persistence of an anastrozole-containing polymeric microsphere prepared by the inventive method, in which the anastrozole-containing polymeric microsphere with each composition, prepared by the inventive method, was intramuscularly injected into a rat (in an amount of 20mg/kg), and the concentration of anastrozole in blood was measured.

[Mode for Invention]

Hereinafter, the present invention will be described in detail with reference to the Examples.
However, Examples as described below are only for illustrative purposes and are not intended to limit the scope of the invention.

**<Example 1> (not in the scope of the claims)**

**Comparison of concentrations of a remaining solvent according to concentrations of a water-insoluble organic solvent mixed with a dispersion solvent**

**<1-1> Measurement of a remaining solvent in the preparation of a** polymeric microsphere **according to a conventional method**

0.25g of 7525 2.5E PLGA polymer was dissolved in 4ml of ethyl formate (EF), and then emulsified in 40ml of 0.5% polyvinyl alcohol (PVA) so as to provide an emulsion. Then, 3.4ml of 28% NH3 solution was added to the emulsion, followed by a reaction for 30 minutes. The resultant product was added with distilled water, and filtered. A microsphere was separated, and then re-dispersed in 80ml of 0.1% PVA, followed by stirring. The information on 7525 2.5E PLGA polymer used in this example is noted in Table 1.

[Table 1]

| Information on 7525 2.5E PLGA polymer | | | |
|---|---|---|---|
| Name and trademark | manufacturer | Lactide : Glycolide | Inherent viscosity |
| 7525DLG2.5E (LAKESHOREBIOMTERIALS) | SurModics Pharmaceuticals Co. (US) | 75 : 25 | 0.25 dL/g |

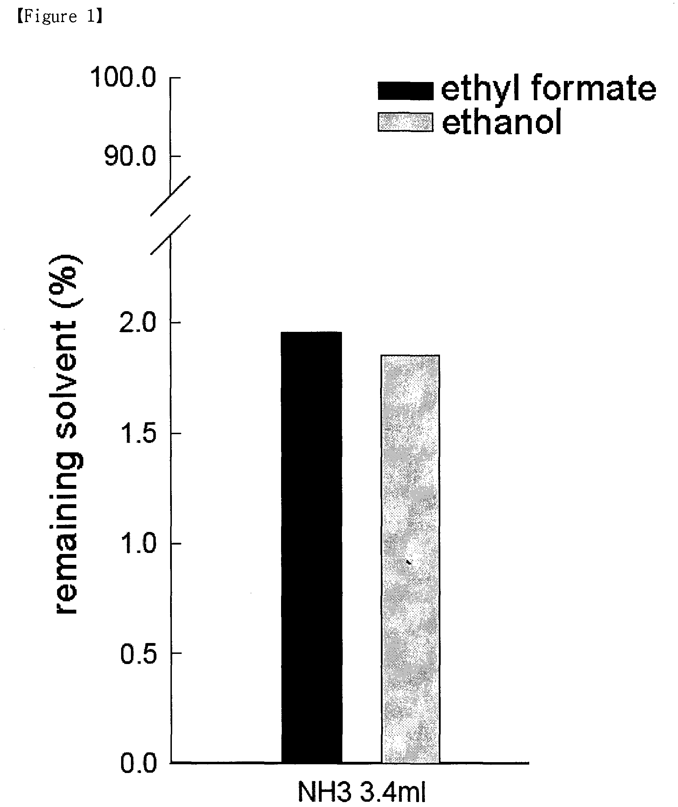

An analysis on a remaining solvent of a microsphere was carried out by gas chromatography (GC) as described below. The GC device was GC-2010 (shimadzu, Japan), and the column was ZB-624 (phenomenex, US). The temperature of SPL was maintained at 200°C, and the sample had a split ratio of 15. As the carrier gas, high purity nitrogen gas was used. The pressure was maintained at 54.3 kPa (flow rate: 1.3ml/min) for 2 minutes, and maintained at a rate of -50°C at 40kPa for 3 minutes. Then, the pressure was raised up to 100kPa at a rate of 80, and maintained for 2 minutes. The temperature of the column was maintained at 80°C for 5.1 minutes, and then raised up to 180°C at a rate of 200°C per min, and maintained for 2 minutes. As a detector, a flame ionization detector (FID) was used, and the temperature was 220°C. About 50mg of a polymeric microsphere sample was collected and the weight was exactly measured. Then, the sample was completely dissolved in 2ml of tetrahydrofuran. The resultant solution was diluted up to 4 times by using pentanol, and the precipitated polymer was filtered, and injected into the GC.
As a result, as shown in FIG. 1, it was found that the remaining solvent was 1.5% or more.

**<1-2> Concentrations of a remaining solvent according to concentrations of a water-insoluble organic solvent mixed with a dispersion solvent**

The concentration of an organic solvent remaining in a polymeric microsphere was measured according to whether or not a dispersion solvent is previously mixed with a water-insoluble organic solvent
A polymeric microsphere was prepared according to polymeric microsphere preparation conditions noted in Table 2 below.

[Table 2]

| polymeric microsphere preparation conditions | | | | | |
|---|---|---|---|---|---|
| No | Amount of organic solvent (EF) mixed with dispersion solvent | Amount of drug (olanzapine ) | Amount of NaOH | Amount of remaining EF (%) | Amount of remaining ethanol (%) |
| A | 0ml | 0mg | 5ml | 0.62 | 0.48 |
| B | 0.5ml | 0mg | 5.6ml | 0.34 | 0.52 |

(continued)

| No | Amount of organic solvent (EF) mixed with dispersion solvent | Amount of drug (olanzapine ) | Amount of NaOH | Amount of remaining EF (%) | Amount of remaining ethanol (%) |
|---|---|---|---|---|---|
| | polymeric microsphere preparation conditions | | | | |
| C | 1.0ml | 0mg | 6.2ml | 0.16 | 0.46 |
| D | 0ml | 60mg | 5ml | 0.55 | 0.94 |
| E | 0.5ml | 60mg | 5.6ml | 0.31 | 1.03 |
| F | 1.0ml | 60mg | 6.2ml | 0.21 | 0.87 |

First, 0.35g of 7525 2.5E polylactide-co-glycolide (PLGA) polymer and olanzapine according to Table 1 were dissolved in 4ml of ethyl formate (EF), and emulsified in 20ml of 0.5 % Poly(vinyl Alcohol) (PVA) previously added with EF organic solvent according to Table 1 so as to provide an emulsion. 10 N NaOH was added to the emulsion, followed by a reaction for 30 minutes. The resultant product was added with distilled water, and filtered. A microsphere was separated, and then re-dispersed in 80ml of 0.1% PVA, followed by stirring.

As a result, as noted in Table 2, it was found that when the dispersion solvent is added with an organic solvent, the concentration of the remaining organic solvent is reduced.

<Example 2> (not in the scope of the claims)

Comparison of concentrations of a remaining solvent according to the temperature of a dispersion solvent in a re-dispersion step

In the preparation of a polymeric microsphere, it was expected that the reaction of acid or base for removing a water-insoluble organic solvent changes the temperature of an aqueous phase, thereby having an effect on the amount of a remaining solvent. Accordingly, the polymeric microsphere preparation process was divided into two steps. In the first step, a base or acid solution is added and the reaction is completed, and in the second step, after the completion of the reaction, a microsphere is filtered and separated, and then is re-dispersed in PVA solution, followed by stirring. In this example, it was determined which step has a temperature as an important role for reducing the remaining solvent.

<2-1> Comparison of concentrations of a remaining solvent according to the temperature of a dispersion solvent in a re-dispersion step in the polymeric microsphere preparation using 7525 2.5E PLGA

The polymeric microsphere preparation process is carried out as described below. 60mg of olanzapine and 250mg of 7525 2.5E PLGA were dissolved in 4ml of ethyl formate. The dispersed phase was emulsified in 20ml of 0.5% PVA aqueous solution having 1ml of ethyl formate previously dissolved therein so as to provide an emulsion. Then, 6.2ml of ION NaOH was added to the emulsion, followed by a reaction for 30minutes so as to provide a polymeric microsphere. The polymeric microsphere was separated, and then re-dispersed in 80ml of 0.1% PVA, followed by stirring. Then, the polymeric microsphere was separated and vacuum-dried.

In the process, the first and second steps were performed, respectively, under the temperature conditions noted in Table 3 below, and the remaining solvents were measured according to the method as described in Example 1.

[Table 3]

| Concentrations of a remaining solvent according to the preparation conditions in the polymeric microsphere preparation using 7525 2.5E PLGA | | |
|---|---|---|
| Preparation condition | Amount of remaining EF(%) | Amount of remaining EtOH(%) |
| Step 1: heated up to 33 °C Step 2: heated up to 33 °C | 0.12 | 0.33 |
| Step 1: not heated Step 2: heated up to 33 °C | 0.09 | 0.36 |
| Step 1: heated up to 33 °C | 0.10 | 0.68 |

(continued)

| Concentrations of a remaining solvent according to the preparation conditions in the polymeric microsphere preparation using 7525 2.5E PLGA | | |
|---|---|---|
| Preparation condition | Amount of remaining EF(%) | Amount of remaining EtOH(%) |
| Step 2: not heated | | |

As a result, as noted in Table 3, when in the second step, heating is not carried out, the amount of remaining ethanol within a polymeric microsphere is greater than 0.5%. On the other hand, when the aqueous phase is maintained at 33°C in the second step, the amount of remaining ethanol was reduced to less than 0.5%. Meanwhile, as long as the temperature of the aqueous phase in the second step was maintained at 33°C, there is no significant difference in the concentration of the remaining solvent between the cases where the temperature in the first step was maintained at 33°C, and the temperature was not maintained.

Accordingly, it was determined that the control of the temperature of an aqueous phase in the step performs an important role of reducing the remaining solvent.

**<2-2> Comparison of concentrations of a remaining solvent according to the temperature of a dispersion solvent in a re-dispersion step in the** polymeric microsphere **preparation using 6535 4.5A PLGA**

In general, as the molecular weight of PLGA is increased, the amount of the organic solvent remaining in the polymeric microsphere is increased. Accordingly, it was tested whether the problem of a remaining organic solvent can be overcome in a case where 6535 4.5A PLGA having a high molecular weight is used in the inventive method.

The information on the PLGA polymer used in the Example is noted in Table 4.

[Table 4]

| Information on 6535 4.5A PLGA polymer | | | |
|---|---|---|---|
| Name and trademark | manufacturer | Lactide : Glycolide | Inherent viscosity |
| 6535DLG4.5A (LAKESHORE BIOMTERIALS) | SurModics Pharmaceuticals Co. (US) | 64 : 36 | 0.49 dL/g |

The process of preparing a polymeric microsphere is the same as that described in Example 2-1 except that as a polymer, 6535 4.5A PLGA was used, instead of 7525 2.5E PLGA.

In the process, the first and second steps were carried out, respectively, under the temperature conditions as noted in Table 5 below, and then the remaining solvent was measured in the same manner as that described in Example 1.

Also, the state of the prepared polymeric microsphere was observed by an electron microscope (SEM, Hitachi S-3000N, Japan, HV 20kv, Working Distance 15mm, Beam current 40).

[Table 5]

| Concentrations of a remaining solvent according to the preparation conditions in the polymeric microsphere preparation using 6535 4.5A PLGA | | |
|---|---|---|
| Preparation condition | Amount of remaining EF (%) | Amount of remaining EtOH (%) |
| Step 1: heated up to 33°C Step 2: heated up to 33°C | 0.08 | 0.55 |
| Step 1: not heated Step 2: not heated | 0.26 | 1.12 |

As a result, as noted in Table 5, even in a case where 6535 4.5A was used, it was found that when the temperature of the aqueous phase in the second step was maintained at 33°C, the amounts of remaining ethyl formate and ethanol were significantly reduced.

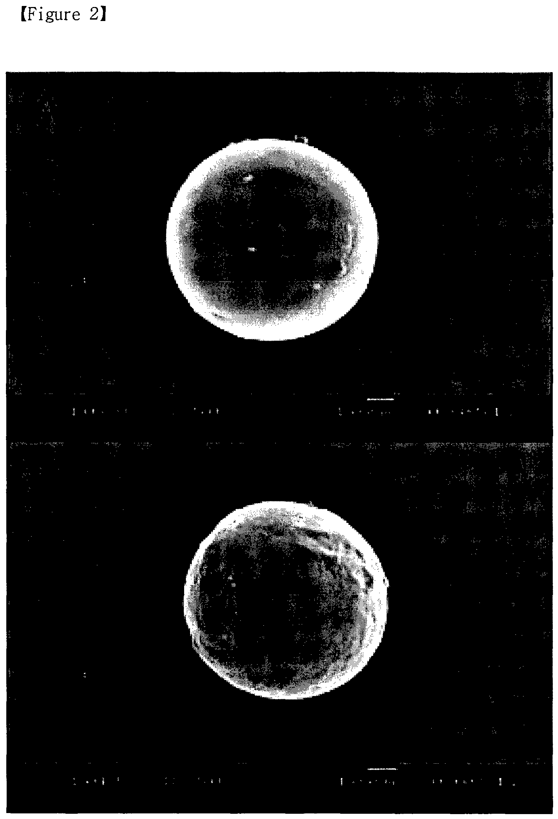

Also, when the polymeric microsphere was photographed by an electron microscope, it was found that the polymeric microsphere having 60mg of olanzapine sufficiently loaded therein was prepared (see FIG. 2).

**<2-3> Comparison of concentrations of a remaining solvent according to the temperatures of a dispersion solvent in the re-dispersion step in a case where the use amount of a polymer is increased**

It was tested whether the concentration of a solvent remaining in a polymeric microsphere can be reduced according to a temperature change of a dispersion solvent even in a case where the concentration of PLGA in the dispersed phase is increased.

60mg or 80mg of olanzapine and 400mg of 6535 4.5A PLGA were dissolved in 4ml of ethyl formate so as to provide a dispersed phase. Then, a polymeric microsphere was prepared in the same manner as that described in Example 2-1, and the concentration of the remaining solvent was measured in the same manner as that described in Example 1.

In the process, the temperature in the second step is noted in Table 6.

[Table 6]

| Concentrations of a remaining solvent according to the preparation conditions in the polymeric microsphere preparation using 6535 4.5A PLGA | | | | |
|---|---|---|---|---|
| batch | Temperature (°C) in the second step | Amount of drug (mg) | Amount of remaining ethyl formate % | Amount of remaining ethanol % |
| 1 | 33 | 60 | 0.91 | 0.13 |
| 2 | 33 | 80 | 1.43 | 0.1 or less |
| 3 | 40 | 60 | 0.1 or less | 0.1 or less |
| 4 | 40 | 80 | 0.1 or less | 0.1 or less |
| 5 | 50 | 60 | 0.1 or less | 0.1 or less |
| 6 | 50 | 80 | 0.1 or less | 0.1 or less |

As a result, as noted in Table 6, it was determined that it is possible to maintain a low concentration of a remaining solvent when the temperature in the second step was maintained at 40°C or more even in a case where the amount of PLGA was increased up to 400mg.

Also, the state of polymeric microspheres in batches 3 and 4 noted in Table 6 was photographed by an electron microscope (SEM, Hitachi S-3000N, Japan, HV 20kv, Working Distance 15mm, Beam current 40).

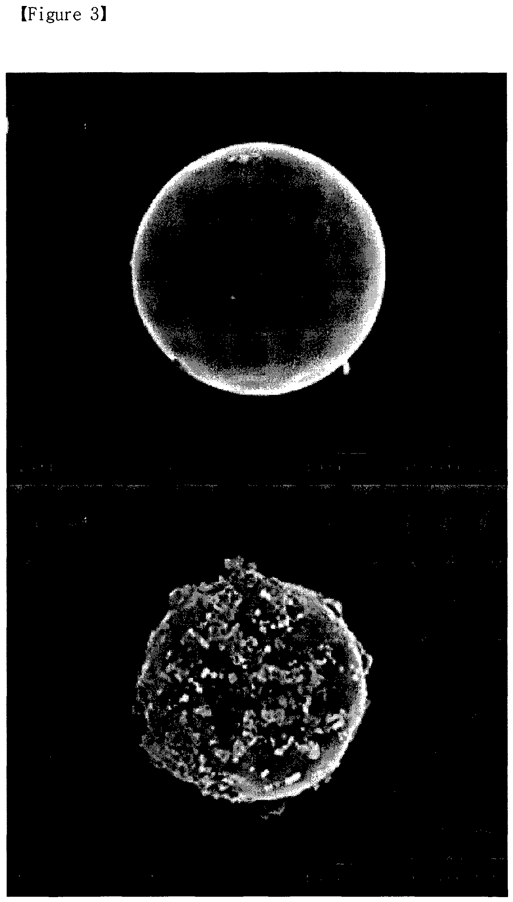

As a result, it was found that a polymeric microsphere having olanzapine loaded therein was prepared (see FIG. 3).

**<Example 3> (not in the scope of the claims)**

**Comparison 1 of concentrations of a remaining solvent according to the concentrations of a water-insoluble organic solvent mixed with a dispersion solvent**

In Example 1, it was found that when an organic solvent is previously added to a dispersion solvent, the concentration of the remaining organic solvent is reduced. Accordingly, while the temperature of a dispersion solvent in a re-dispersion step is maintained at a high temperature, the amounts of a remaining solvent were measured according to the varying amounts of a water-insoluble organic solvent mixed with a dispersion solvent.

500mg of 4.5A PLGA (Lactide: Glycolide=85:15, 0.45 dL/g, SurModics Pharmaceuticals Co., US) was dissolved in 4ml of ethyl formate so as to provide a dispersed phase. The dispersed phase was emulsified in 20ml of 0.5% PVA aqueous solution so as to provide an emulsion, in which the PVA aqueous solution was previously added with 0, 1, 2 or 4ml of ethyl formate. Then, 6.2ml of 10N NaOH was added to the emulsion, followed by a reaction for 30 minutes, so as to provide a polymeric microsphere. The polymeric microsphere was separated, and then re-dispersed in 80ml of 0.1% PVA at 40°C, followed by stirring. Then, the polymeric microsphere was separated and vacuum-dried.

After the preparation of the polymeric microsphere, the remaining solvent was measured in the same manner as that described in Example 1, and the prepared polymeric microsphere was photographed in the same manner as that described in Example 2-2.

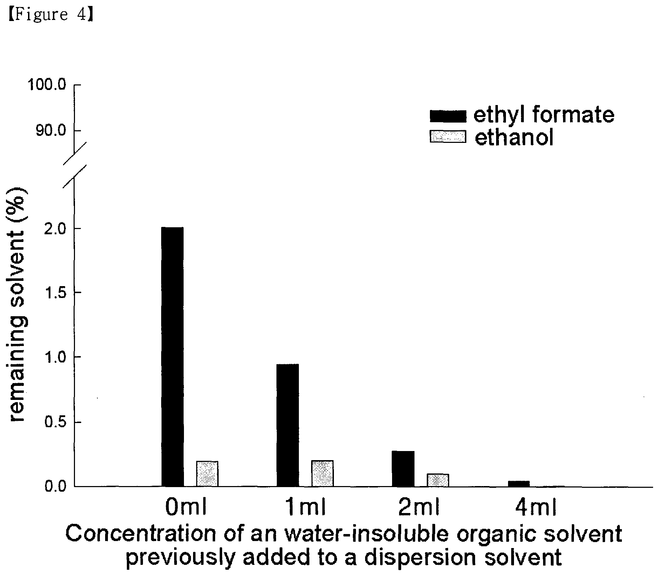

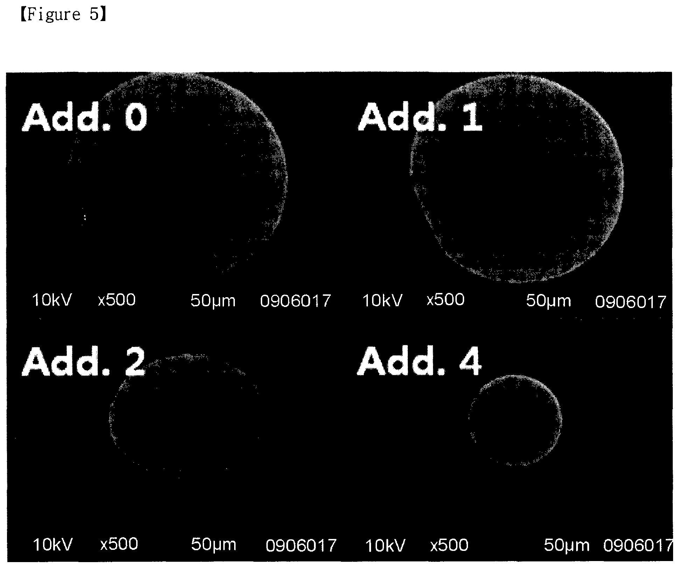

As a result, as shown in FIG. 4, it was found that as the concentration of an organic solvent previously added to a dispersion solvent is increased, the concentration of a solvent remaining in a polymeric microsphere is reduced. Also, as shown in FIG. 5, although an organic solvent is previously added to a dispersion solvent, it is possible to stably prepare a spherical polymeric microsphere. Furthermore, it was found that as the concentration of the organic solvent previously added to the dispersion solvent is increased, the size of the prepared polymeric microsphere is reduced.

**<Example 4> (not in the scope of the claims)**

**Comparison of concentrations of a remaining solvent according to a ratio of a dispersed phase to a dispersion solvent**

**<4-1> Preparation of a polymeric microsphere according to a change of a ratio of a dispersed phase to a dispersion solvent**

According to a change of a ratio of a dispersed phase to a dispersion solvent, in order to determine a change of the amount of a remaining solvent, a polymeric microsphere was prepared while the ratio of the dispersed phase (0) to the dispersion solvent (W) is adjusted to 1:10, 1:6 or 1:4 as noted in Table 7. The ratio of the dispersed phase to the dispersion solvent was adjusted by adjusting the amount of the dispersed phase or the amount of the dispersion solvent. Also, as an organic solvent constituting the dispersed phase, ethyl acetate or ethyl formate was used, and as a solvent decomposition reagent, NaOH or ammonia was used.

[Table 7]

| A ratio of 0/W used in the test and a solvent decomposition reagent | | | | |
|---|---|---|---|---|
| | O/W ratio | Dispersed phase(ml) | Dispersion solvent (ml) | Solvent decomposition reagent |
| EA-1 | 1:4 | 40 | 160 | NaOH |
| EA-2 | 1:6 | 27 | 160 | NaOH |
| EA-3 | 1:10 | 16 | 160 | NaOH |
| EA-4 | 1:6 | 27 | 160 | NaOH |
| EA-5 | 1:4 | 16 | 64 | NaOH |
| EA-6 | 1:6 | 16 | 96 | NaOH |
| EF-1 | 1:4 | 42.5 | 170 | NH3 |
| EF-2 | 1:6 | 28.3 | 24 | NH3 |
| EF-3 | 1:10 | 17 | 170 | NH3 |
| EF-4 | 1:4 | 17 | 68 | NH3 |
| EF-5 | 1:6 | 17 | 102 | NH3 |
| EF-6 | 1:4 | 40 | 160 | NaOH |
| EF-7 | 1:6 | 27 | 160 | NaOH |
| EF-8 | 1:10 | 16 | 160 | NaOH |
| EF-9 | 1:4 | 16 | 64 | NaOH |
| EF-10 | 1:6 | 16 | 96 | NaOH |

A polymer compound blend 1g (4A:2A = 6:4) and olanzapine 0.333g were introduced, and an equivalent amount of ethyl acetate or ethyl formate was added thereto. The mixture was completely dissolved, and an equivalent amount of 0.5% PVA was added thereto, followed by stirring at room temperature. An equivalent amount of 10M NaOH or ammonia solution was added thereto, followed by stirring for 30 minutes so as to provide a polymeric microsphere. The prepared polymeric microsphere was filtered and collected, and re-dispersed in 0.1% PVA, followed by stirring. Then, the resultant product was washed with distilled water and freeze-dried.

**<4-2> Measurement of the yield of a prepared polymeric microsphere, and the remaining solvent**

The remaining solvent of the polymeric microsphere prepared from Example 4-1 was measured in the same manner as that described in Example 1.
The collected polymeric microsphere was placed on a plate (whose weight was previously measured), and then its weight after vacuum-drying was measured. Then, the yield of the polymeric microsphere was calculated by the equation below.

$$\text{Yield (\%)} = \text{(weight of collected polymeric microsphere)/(sum of weights of polymer and drug used in the preparation)} \times 100$$

[Table 8]

| The yield of a prepared polymeric microsphere, and the amount of a remaining solvent | | | |
|---|---|---|---|
| | Amount of remaining solvent (%) | | yield (%) |
| | EA | EtOH | |
| EA-1 | 0.023 | 1.273 | 62.5 |
| EA-2 | 0.058 | 1.249 | 38.9 |
| EA-3 | 1.289 | 0.180 | 79.8 |
| EA-4 | 0.113 | 0.997 | 53.9 |
| EA-5 | 0.057 | 0.506 | 54.5 |
| EA-6 | 0.133 | 0.366 | 68.6 |
| EF-1 | 0.249 | 0.199 | 66.8 |
| EF-2 | 0.048 | 0.027 | 26.3 |
| EF-3 | 1.486 | 0.136 | 92.6 |
| EF-4 | 0.530 | 0.070 | 77.9 |
| EF-5 | 0.210 | N.D. | 62.0 |
| EF-6 | N.D. | 1.281 | 43.5 |
| EF-7 | 0.020 | 0.192 | 44.3 |
| EF-8 | 0.365 | N.D. | 71.0 |
| EF-9 | 0.227 | 0.714 | 52.2 |
| EF-10 | 0.739 | 0.562 | 59.5 |

As a result, as noted in Table 8, when the ratio of the dispersed phase and the dispersion solvent is 1:4 or 1:6, the amount of a remaining solvent (EA or EF) is reduced, compared to that in 1:10. However, it was found that the amount of remaining ethanol is increased. Thus, it was found that it doesn't matter which one of the dispersed phase and the dispersion solvent in the ratio of the dispersed phase and the dispersion solvent was adjusted.

<Example 5>

The change of a polymeric microsphere according to the ratio of a dispersed phase to a dispersion solvent, the concentration of a previously added organic solvent, and a temperature change in a re-dispersion step

<5-1> polymeric microsphere preparation and measurement of a remaining solvent

The concentration of a remaining solvent was measured according to the concentration change of a water-insoluble organic solvent mixed with a dispersion solvent, the temperature change of a dispersion solvent in the re-dispersion step (in the second step), and the change of a ratio of a dispersed phase and the dispersion solvent in Examples 1 to 4.

The polymeric microsphere was prepared in consideration of these characteristics, and the concentration of the remaining solvent was measured.

As a dispersed phase, a solution including 250mg of 4.5A PLGA dissolved in 4ml of ethyl formate was used.

As a dispersion solvent, 20, 30, or 40ml of 0.5% PVA was used, and a water-insoluble organic solvent (ethyl formate) was previously added to the dispersion solvent in an amount ranging from 0 to 4ml according to the amount of the dispersion solvent. For the decomposition of the solvent, ION NaOH was used, which was added in consideration of a total amount of added ethyl formate in a 1:1 equivalent amount. In the preparation of the polymeric microsphere, the first step for adding a base or acid solution and carrying out the reaction was carried out at room temperature, and after the reaction, the second step for re-dispersing the polymeric microsphere filtered and separated in a PVA solution and stirring the mixture was carried out at 40°C.

The analysis of the remaining solvent of the prepared polymeric microsphere was measured in the same manner as that described in Example 1. The preparation process and the remaining solvent analysis were repeatedly carried out twice, and all of the measured values were noted.

[Table 9]

| The amount of a remaining solvent of a prepared polymeric microsphere | | | | | | |
|---|---|---|---|---|---|---|
| First step at room temperature (RT) | Amount of dispersion solvent (ml) | Amount of EF previously added to dispersion solvent (ml) | Remaining EF (%) | | Remaining ethanol (%) | |
| | | | #1 | #2 | #1 | #2 |
| | 20 | 0 | 0.175 | 0.113 | 0.063 | 0.080 |
| | | 1 | 0.053 | 0.015 | 0.044 | 0.049 |
| | | 2 | 0.036 | 0.006 | 0.044 | 0.056 |
| | 30 | 0 | 0.285 | 0.291 | 0.038 | 0.052 |
| | | 1 | 0.156 | 0.119 | 0.056 | 0.052 |
| | | 2 | 0.059 | 0.028 | 0.037 | 0.032 |
| | | 3 | 0.042 | 0.020 | 0.078 | 0.041 |
| | 40 | 0 | 0.932 | 1.204 | 0.058 | 0.105 |
| | | 1 | 0.231 | 0.225 | 0.038 | 0.053 |
| | | 2 | 0.164 | 0.079 | 0.040 | 0.043 |
| | | 3 | 0.111 | 0.061 | 0.055 | 0.042 |
| | | 4 | 0.040 | 0.038 | 0.023 | 0.112 |

As a result, as noted in Table 9, compared to the case where no ethyl formate was added to the dispersion solvent (comparative compositions), the addition of ethyl formate reduced the concentration of the remaining solvent. Especially, the amount of the remaining solvent was increased when a ratio of the dispersed phase to the dispersion solvent is 1:10 (40ml of dispersion solvent PVA) and the dispersion solvent was not previously added with EF.

**<5-2> Measurement of the yield of a polymeric microsphere**

The yield of the polymeric microsphere obtained from Example 5-1 was measured. The measurement was carried out in the same manner as that described in Example 4-2.

As a result, as noted in Table 10, it was found that under all conditions, there is no problem in the yield of the polymeric microsphere.

[Table 10]

| Yield of the prepared polymeric microsphere | | | | |
|---|---|---|---|---|
| First step at room temperature (RT) | Amount of dispersion solvent PVA (ml) | Amount of EF previously added to dispersion solvent (ml) | Yield (%) | |
| | | | #1 | #2 |
| | 20 | 0 | 79.56 | 70.24 |
| | | 1 | 66.48 | 63.88 |
| | | 2 | 76.68 | 66.52 |
| | 30 | 0 | 69.96 | 70.24 |
| | | 1 | 40.04 | 75.76 |
| | | 2 | 77.56 | 74.64 |
| | | 3 | 88.28 | 74.16 |
| | 40 | 0 | 67.68 | 72.4 |
| | | 1 | 67.28 | 82.8 |
| | | 2 | 39.6 | 82.2 |
| | | 3 | 77.04 | 79.64 |
| | | 4 | 55.96 | 77.92 |

**<5-3> Measurement (GPC) of a molecular weight change of 4.5A PLGA before/after the preparation of a polymeric microsphere**

In the preparation process of the polymeric microsphere in Example 5-1, in order to determine a molecular weight change of PLGA, the molecular weight of 4.5A PLGA before/after the preparation of the polymeric microsphere was measured through gel permeation chromatography (GPC).

In the GPC, Agilent 1100 (Agilent Technologies, Inc, US) was used, and as a mobile phase, tetrahydrofuran was used. As an analysis column, PLgel 5um 100Å 79911GP-504 (Agilent) was used, and the flow rate was maintained at 1ml/min. In order to obtain a standard curve, a standard product (Polymer Laboratories, molecular weight: 3390000, 1290000, 426600, 151700, 72200, 28500, 9860, 4950, 1300) was used. As the analysis sample, 8mg of a polymeric microsphere was used, which was dissolved in 2ml of tetrahydrofuran and filtered. The amount injected into the GPC was made 50ul.

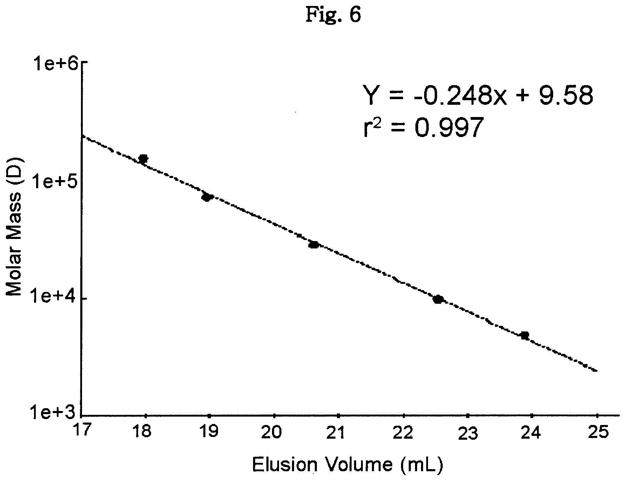

By using the standard product, the standard curve shown in FIG. 6 was obtained. Based on this, the molecular weights of 4.5A PLGA before the preparation, and 4.5A PLGA of the prepared polymeric microsphere were measured. As a result, as noted in Table 11, the molecular weight of 4.5A PLGA of the polymeric microsphere prepared by the inventive method ranged from 55421 to 58409. Thus, there was no significant change compared to the before-preparation molecular weight (57542) of 4.5A.

Accordingly, it was found that in the inventive polymeric microsphere preparation method, the polymer of 4.5A PLGA is not decomposed.

[Table 11]

| Comparison of molecular weights of 4.5A PLGA | | | | |
|---|---|---|---|---|
| First step at room temperature (RT) | Amount of solvent PVA | Amount of EF previously added to dispersion solvent (ml) | GPC | |
| | | | Mw | polydisper sity |
| | 20 | 0 | 57901 | 1.4594 |
| | | 1 | 57294 | 1.5004 |
| | | 2 | 55743 | 1.5865 |
| | 30 | 0 | 57498 | 1.5405 |
| | | 1 | 55573 | 1.6392 |
| | | 2 | 57486 | 1.5552 |
| | | 3 | 55421 | 1.649 |
| | 40 | 0 | 57788 | 1.4495 |
| | | 1 | 57704 | 1.5207 |
| | | 2 | 57994 | 1.5454 |
| | | 3 | 57423 | 1.4922 |
| | | 4 | 58409 | 1.4399 |
| Before preparation of microsphere, 4.5A PLGA | | | 57542 | 1.6122 |

<Example 6>

Measurement of a molecular weight change of a polymer compound of a drug-containing polymeric microsphere

It was found that in Example 5-3, when a drug is not contained, there is no change in the molecular weight of the polymeric microsphere. Then, a polymeric microsphere containing a drug was prepared under various conditions, and the molecular weight of the polymer compound was measured.

[Table 12]

| Preparation conditions of a polymeric microsphere | | | | | | | | |
|---|---|---|---|---|---|---|---|---|
| Test no. | Kind of polyme r | Amount of polymer (mg) | Kind of adrug | Content of adrug | Organic solvent | Amount of organic solvent | Temperature in first step | Temperature in second step |
| 1 | 5050 2A | 1000 | Anastrozo le | 111mg | Ethyl formate | 27 | 33 | 40 |
| 2 | 7525 2.5E | 1000 | Anastrozo le | 667mg | Ethyl formate | 19 | 4 | 33 |
| 3 | 6535 4.5A | 250 | olanzapin e | 60mg | Ethyl formate | 4 | 4 | 40 |

A dispersed phase was prepared according to Table 12, and was emulsified in 0.5% PVA so as to provide an emulsion. Then, 10N NaOH solution was added to the emulsion, followed by a reaction for 30 minutes. The resultant product was added with distilled water, and a polymeric microsphere was filtered and separated. The separated polymeric microsphere was re-dispersed in 0.1% PVA solution, followed by vacuum-drying.-In the preparation of the polymeric microsphere, the first step for adding a base or acid solution and carrying out the reaction was carried out, and after the reaction, the second step for re-dispersing the polymeric microsphere filtered and separated in a PVA solution and stirring the mixture was carried out. The temperatures of the respective steps were maintained as noted in Table 12.

The molecular weight of the polymer compound of the prepared polymeric microsphere was measured in the same manner as that described in Example 5-3.

As a result, as noted in Table 13, it was found that the molecular weights of the polymer compounds of all polymeric microspheres can be sufficiently maintained. Thus, it was determined that in the inventive preparation method, a molecular weight of a polymer compound can be well maintained irrespective of conditions such as the kind of a drug, the reaction temperature.

[Table 13]

| Measurement result of a molecular weight of a polymer compound before/after polymeric microsphere preparation | | |
|---|---|---|
| Test No | Molecular weight before microsphere preparation | Molecular weight after microsphere preparation |
| 1 | 15000 | 15709 |
| 2 | 29013 | 27737 |
| 3 | 56694 | 54116 |

**<Example 7>**

**Preparation of a polymeric microsphere containing olanzapine**

**<7-1> Preparation of a polymeric microsphere containing olanzapine**

A polymeric microsphere containing olanzapine was prepared under the condition noted in Table 14 according to the inventive polymeric microsphere preparation method.

60mg of olanzapine and 250mg of 6535 4.5A PLGA were dissolved in 4ml of ethyl formate. The dispersed phase was emulsified in a dispersion solvent (0.5% PVA) previously added with ethyl formate according to Table 14, so as to provide an emulsion. Then, 10N NaOH solution was added to the emulsion, followed by a reaction for 30 minutes. The resultant product was added with distilled water, and a polymeric microsphere was filtered and separated. The separated polymeric microsphere was re-dispersed in 0.1% PVA solution, followed by vacuum-drying. In the preparation of the polymeric microsphere, the first step for adding a base or acid solution and carrying out the reaction was carried out at room temperature, and after the reaction, the second step for re-dispersing the polymeric microsphere filtered and separated in a PVA solution and stirring the mixture was carried out at 40°C.

[Table 14]

| Preparation conditions of a polymeric microsphere containing olanzapine | | | | | | |
|---|---|---|---|---|---|---|
| Temperature of first step | Kind of PLGA | Amount of dispersion solvent PVA (ml) | Amount of EF previously added to dispersion solvent (ml) | olanzapine (mg) | Temperature of second step | NaOH for Solvent decomposition |
| Room temperature (RT) | 6535DLG4.5A | 30 | 2 | 60 | 40 | 7.4 |
| | | | 3 | 60 | 40 | 8.6 |
| | | 40 | 3 | 60 | 40 | 8.4 |
| | | | 4 | 60 | 40 | 9.6 |

**<7-2> Measurement of the amount of a remaining solvent, the loading efficiency of olanzapine, and the yield of a polymeric microsphere**

The amount of the remaining solvent of the polymeric microsphere prepared from Example 7-1 was measured in the same manner as that described in Example 1.

The prepared polymeric microsphere was placed on a dried vessel, and its weight after freeze-drying was measured. Then, the yield of the polymeric microsphere was calculated by the equation below.

<513> Yield (%) = (weight of polymeric microsphere)/(sum of weights of polymer and drug used in the preparation) X 100

In the loading efficiency of olanzapine, the loading amount and the loading efficiency of the prepared polymeric microsphere were measured as described below.

10mg of a dried polymeric microsphere was dissolved in a solution of acetonitrile: water =50:50(v/v), and was diluted a proper number of times. The resultant solution was filtered, and analyzed by UPLC (Ultra Performance Liquid Chromatography). Then, the loading amount and the loading efficiency were calculated. As an UPLC device, ACQUITY (Waters, Germany) was used, and as a column, HSS C18 (Waters ACQUITY UPLC, Japan) was used. A mobile phase included ammonium acetate buffer and acetonitrile in a mixed ratio of 50:50, and a dilution solution included acetonitrile and water in a mixed ratio of 50:50.

<516> Loading amount (%) = (weight of detected drug /weight of microsphere) X 100

Loading efficiency (%) = loading amount /(theoretical loading amount) X 100

theoretical loading amount (%) = (weight of drug used in the preparation)/( sum of weights of polymer and drug used in the preparation) X 100

As a result, it was found that the small amount of the remaining solvent was maintained as noted in Table 15, and almost all the loading efficiencies and the yields showed a high value of 70% or more as noted in Table 16.

[Table 15]

| Amount of a remaining solvent of a prepared polymeric microsphere | | | | | | |
|---|---|---|---|---|---|---|
| Temperature of first step | Amount of dispersion solvent PVA (ml) | Amount of EF added to dispersion solvent (ml) | Remaining EF(%) | | Remaining ethanol (%) | |
| | | | #1 | #2 | #1 | #2 |
| Room temperature (RT) | 30 | 2 | 0.023 | 0.006 | 0.199 | 0.163 |
| | | 3 | 0.013 | 0.003 | 0.168 | 0.116 |
| | 40 | 3 | 0.022 | 0.011 | 0.208 | 0.140 |
| | | 4 | 0.013 | 0.014 | 0.201 | 0.145 |

[Table 16]

| Loading efficiency and yield of the prepared polymeric microsphere | | | | | | |
|---|---|---|---|---|---|---|
| Temperature in first step | Amount of dispersion solvent PVA (ml) | Amount of EF added to (ml)dispersion solvent (ml) | Loading efficiency (%) | | Yield (%) | |
| | | | #1 | #2 | #1 | #2 |
| Room temperature (RT) | 30 | 2 | 80.719 | 76.789 | 73.613 | 72.616 |
| | | 3 | 80.215 | 69.635 | 74.226 | 72.958 |
| | 40 | 3 | 80.858 | 74.364 | 71.613 | 75.723 |
| | | 4 | 74.205 | 68.040 | 72.613 | 75.942 |

**<Example 8>**

**Preparation of an olanzapine-containing polymeric microsphere by using ethyl acetate as a water-insoluble organic solvent**

**<8-1> Preparation of an olanzapine-containing polymeric microsphere**

An olanzapine-containing polymeric microsphere was prepared by using another water-insoluble organic solvent than the water-insoluble organic solvent (ethyl formate) used in Example 7.

In this Example, as the water-insoluble organic solvent, ethyl acetate (EA) was used, and the polymeric microsphere was prepared under the preparation conditions noted in Table 17.

60mg of olanzapine and 250mg of 4.5A PLGA were dissolved in 4ml of ethyl acetate so as to provide a dispersed phase. The dispersed phase was emulsified in a dispersion solvent (0.5% PVA) previously added with ethyl acetate according to Table 17 so as to provide an emulsion. Then, 10N NaOH solution was added to the emulsion, followed by a reaction for 30minutes. The resultant product was added with distilled water, and a polymeric microsphere was filtered and separated. The separated polymeric microsphere was re-dispersed in 0.1% PVA solution, followed by vacuum-drying. In the preparation of the polymeric microsphere, the first step for adding a base or acid solution and carrying out the reaction was carried out at 4°C, and after the reaction, the second step for re-dispersing the polymeric microsphere filtered and separated in a PVA solution and stirring the mixture was carried out at 40 °C.

[Table 17]

| Preparation conditions of a polymeric microsphere containing olanzapine | | | | | | |
|---|---|---|---|---|---|---|
| Temperature of first step | Kind of PLGA | Amount of dispersion solvent PVA (ml) | Amount of EA dispersion previously added to dispersion solvent (ml) | olanzapine (mg) | Temperature of second step | NaOH (ml) for Solvent decomposition |
| 4 | 6535 4.5A | 30 | 2 | 60 | 40 | 7.4 |
| | | | 3 | 60 | 40 | 8.6 |
| | | 40 | 3 | 60 | 40 | 8.4 |
| | | | 4 | 60 | 40 | 9.6 |

**<8-2> Measurement of the amount of a remaining solvent, the loading efficiency of olanzapine, and the yield of a polymeric microsphere**

The amount of the remaining solvent of the polymeric microsphere prepared from Example 8-1 was measured in the same manner as that described in Example 1.

As a result, it was found that the small amount of the remaining solvent was maintained as noted in Table 18, and almost all the loading efficiencies and the yields showed high value of 85% or more and 70% or more, respectively, as noted in Table 19.

[Table 18]

| Amount of a remaining solvent of a prepared polymeric microsphere | | | | | | |
|---|---|---|---|---|---|---|
| Temperature of first step | Amount of dispersion solvent PVA (ml) | Amount of EF added to dispersion solvent (ml) | Remaining EA (%) | | Remaining ethanol (%) | |
| | | | #1 | #2 | #1 | #2 |
| 4 | 30 | 2 | 0.151 | 0.153 | 0.513 | 0.410 |
| | | 3 | 0.134 | 0.120 | 0.578 | 0.506 |
| | 40 | 3 | 0.085 | 0.091 | 0.280 | 0.242 |
| | | 4 | 0.129 | 0.130 | 0.310 | 0.458 |

[Table 19]

| Loading efficiency and yield of the prepared polymeric microsphere | | | | |
|---|---|---|---|---|
| Temperatur e of first step | Amount of dispersion solvent PVA | Amount of EF previously added to dispersion solvent (ml) | Loading efficiency (%) | Yield (%) |
| 4 | 30 | 2 | 90.952 | 71.05 |
| | | 3 | 88.892 | 71.45 |
| | 40 | 3 | 85.335 | 75.71 |
| | | 4 | 88.993 | 67.32 |

**<Example 9>**

**A pharmacokinetic test on an olanzapine-containing polymeric microsphere**

**<9-1> Preparation of an olanzapine-containing polymeric microsphere**

According to compositions noted in Table 20, various PLGAs were used so as to prepare an olanzapine-containing polymeric microsphere.

PLGA and olanzapine were dissolved in an organic solvent (ethyl formate) so as to provide a dispersed phase. The ratio of the dispersed phase to a dispersion solvent was 1:5, and as the dispersion solvent, a 10:1 mixture of 0.5% PVA and ethyl formate was used. The dispersed phase was emulsified in the dispersion solvent cooled to 4°C so as to provide an emulsion. Then, 10M NaOH was added to the emulsion as noted in Table 20, followed by a reaction for 30minutes so as to provide a polymeric microsphere. The prepared polymeric microsphere was washed with distilled water, and re-dispersed in 0.1% PVA solution at 40°C, followed by stirring. Then, the polymeric microsphere was separated and vacuum-dried.

The state of the prepared polymeric microsphere was observed by an electron microscope (SEM, Hitachi S-3000N, Japan, HV 20kv, Working Distance 15mm, Beam current 40).

[Table 20]

| Composition of an olanzapine-containing polymeric microsphere | | | | | | |
|---|---|---|---|---|---|---|
| Preparatio n No. | Kind of PLGA | | Organic solvent (ml) | olanzapine (mg) | Amount or NaOH | 0.5% PVA |
| 1 | 8515 | 4.5E | 30 | 667 | 56 | 150 |
| 2 | 100 DL | 2E | 30 | 667 | 56 | 150 |
| 3 | 100 DL | 4.5E | 30 | 667 | 56 | 150 |
| 4 | 100 DL | 2E | 10 | 240 | 18.6 | 50 |

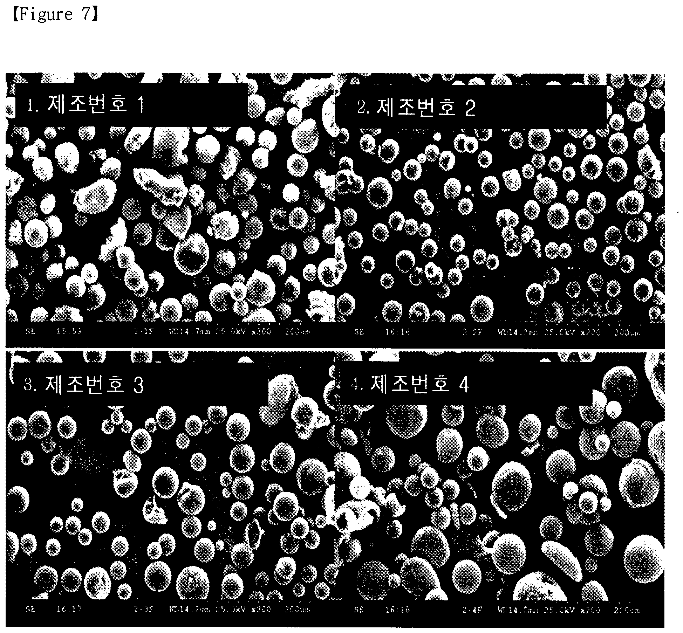

Through the observation by the electron microscope, it was found that olanzapine was well loaded into the polymeric microsphere, and all of the polymeric microspheres were well prepared in a spherical form (see FIG. 7).

**<9-2> test on injectability and dispersibility**

The olanzapine-containing polymeric microsphere prepared from Example 9-1 was suspended at a concentration of 30%(w/v) in a saline solution containing carboxymethyl cellulose (CMC) and tween 20. 1ml of the resultant solution was loaded into a syringe (19G) and then injected so as to determine the injectability.

As a result, as noted in Table 21, it was found that the inventive olanzapine-containing polymeric microsphere was good in injectability and dispersibility.

[Table 21]

| Test result of injectability and dispersibility | |
| --- | --- |
| Preparation No | Test result |
| 1 | + |
| 2 | + |
| 3 | + |

### <9-3> Test on the persistence of an olanzapine-containing polymeric microsphere

The olanzapine-containing polymeric microsphere prepared from Example 9-1 was suspended in an excipient solution (saline including carboxymethyl cellulose and tween 20), and was administered to female SD rats (aged 9 weeks) in an amount of 40mg/kg through intramuscular injection. Then, at a predetermined time interval, the blood was collected, and the concentration of olanzapine in the blood was measured.

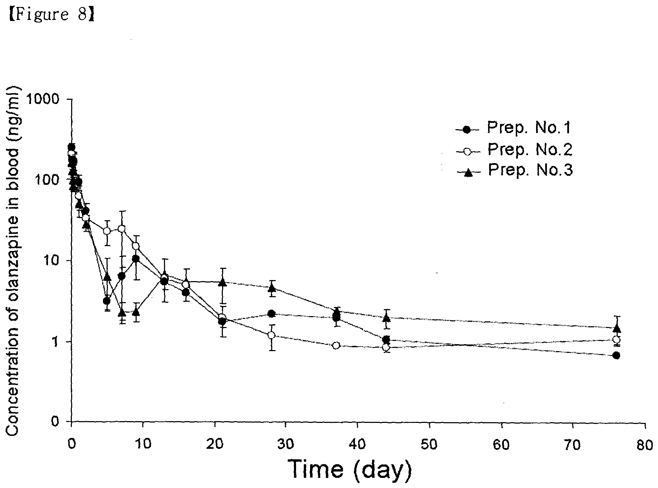

As a result, as shown in FIG. 8, it was found that the inventive olanzapine-containing polymeric microsphere released olanzapine for a maximum of 80 days.

### <Example 10>

### Preparation of an anastrozole-containing polymeric microsphere

According to the inventive polymeric microsphere preparation method, a polymeric microsphere was prepared by using another kind of a polymer, another drug, and another loading amount than those used in the above described Example.

### <10-1> Preparation of an anastrozole-containing polymeric microsphere

According to the formulation noted in Table 22, under various conditions, an anastrozole-containing polymeric microsphere was prepared.

[Table 22]

| Composition of an anastrozole-containing polymeric microsphere | | |
| --- | --- | --- |
| Preparation No. | Kind of PLGA | anastrozole (mg) |
| 1 | 5050-4A | 429 |
| 2 | 7525-2.5E | 429 |
| 3 | 7525-7E | 429 |
| 4 | 8515-3E | 429 |
| 5 | 8515-4.5E | 429 |
| 6 | 100-DL 2E | 429 |
| 7 | 7525-2.5E | 667 |
| 8 | 8515-4.5E | 667 |

Anastrozole and 1000mg of PLGA were completely dissolved in 19ml of ethyl formate so as to provide a dispersed phase. The dispersed phase was emulsified in 0.5% PVA (dispersion solvent) which was cooled at 4°C and added with 8ml of ethyl formate, followed by stirring so as to provide an emulsion. Then, 34ml of 10M NaOH was added to the emulsion, followed by stirring for 30 minutes. The resultant prepared polymeric microsphere was washed with distilled water and re-dispersed in 0.1% PVA at 33°C, followed by stirring. Then, the resultant product was filtered and freeze-dried so as to provide a polymeric microsphere.

**<10-2> Test on basic characteristics of a prepared anastrozole-containing polymeric microsphere**

The prepared polymeric microsphere was placed on a dried vessel, and then its weight after freeze-drying was measured. Then, the yield of the polymeric microsphere was calculated by the equation below.

$$\text{Yield (\%)} = \text{(weight of polymeric microsphere)/(sum of weights of polymer and drug used in the preparation)} \times 100$$

The loading amount and the loading efficiency of the prepared polymeric microsphere were measured as described below.

10mg of a dried polymeric microsphere was dissolved in a solution of acetonitrile: water =50:50(v/v), and was diluted a proper number of times. The resultant solution was filtered, and analyzed by UPLC. Then, the loading amount and the loading efficiency were calculated. As an UPLC device, ACQUITY (Waters, Germany) was used, and as a column, HSS C18 (Waters ACQUITY UPLC, Japan) was used. A mobile phase included ammonium acetate buffer and acetonitrile in a mixed ratio of 50:50, and a dilution solution included acetonitrile and water in a mixed ratio of 50:50.

$$\text{Loading amount (\%)} = \text{(weight of detected drug /weight of microsphere)} \times 100$$

$$\text{Loading efficiency (\%)} = \text{loading amount /(theoretical loading amount)} \times 100$$

$$\text{theoretical loading amount (\%)} = \text{(weight of drug used in the preparation)/( sum of weights of polymer and drug used in the preparation)} \times 100$$

Particle size analysis was carried out by using Malvern mastersizer (Malvern Instruments Ltd, UK).

The amount of the remaining solvent was measured in the same manner as that described in Example 1, and the shape of the polymeric microsphere was photographed in the same manner as that described in Example 2-2.

[Table 23]

| The yield, the loading amount, and the concentration of a remaining solvent of the prepared polymeric microsphere | | | | | | | |
|---|---|---|---|---|---|---|---|
| Preparation No. | yield (%) | Loading amount (%) | Loading efficiency (%) | Average size (um) | Moisture content (%) | remaining EF (%) | Remainin g ethanol (%) |
| 1 | 82.44 | 26.6 | 88.68 | 56.0 | - | N.D. | N.D. |
| 2 | 82.93 | 24.68 | 82.25 | 44.8 | 0.47 | N.D. | N.D. |
| 3 | 98.81 | 25.41 | 84.71 | 61.6 | - | 0.026 | N.D. |
| 4 | 83.55 | 25.88 | 86.26 | 49.9 | 0.46 | N.D. | N.D. |
| 5 | 85.86 | 26.41 | 87.12 | 59.8 | 0.51 | N.D. | N.D. |
| 6 | 81.88 | 25.00 | 83.34 | 41.1 | 0.47 | N.D. | N.D. |
| 7 | 79.48 | 30.41 | 76.04 | 32.5 | - | N.D. | N.D. |
| 8 | 82.42 | 32,97 | 82.42 | 52.7 | - | N.D. | N.D. |

As a result, as noted in Table 23, the yield and the loading efficiency were almost 80% or more, which is appropriate irrespective of the kind of PLGA. Also, the average particle size was found to have an appropriate value ranging from 30 to 60um. The moisture content was about 0.5%, and the remaining solvent was included in a very low amount, which was found to be appropriate irrespective of the kind of a test sample.

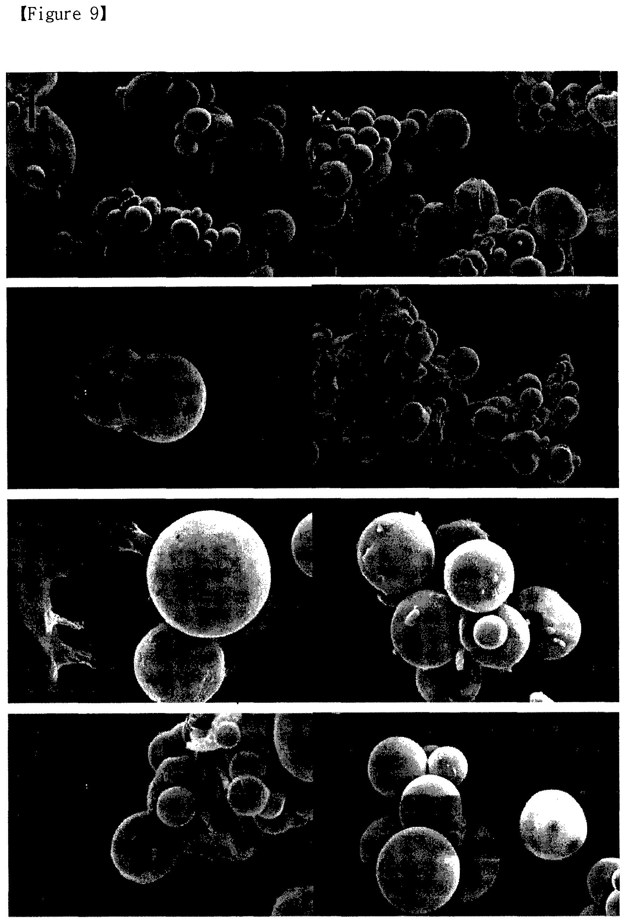

Also, through the photograph by the electron microscope, as shown in FIG. 9, it can be found that the polymeric microsphere was generally well prepared in a smooth spherical shape irrespective of the kind of used PLGA. Especially, it was found that even when the drug content was increased up to 40%, the drug was well loaded and no crystallization of the drug was observed from the surface.

### <10-3> Test on the stability of a prepared anastrozole-containing polymeric microsphere

The analysis on a related compound within a prepared polymeric microsphere was carried out for Preparation No. 8. A polymeric microsphere was weighted in such a manner that API (Active Pharmaceutical Ingredients) anastrozole can be 250ug/ml with respect to the loading amount within the polymeric microsphere, and then was diluted in a solution of acetonitrile: DW = 50:50. Then, it was analyzed by UPLC.

For GPC analysis, 30mg of the prepared polymeric microsphere was dissolved in 3ml of (o-CP: chloroform = 1:3), and was subjected to GPC analysis by an RI detector through a column of PLgel 5$\mu$m Mixed-D*2ea at 40°C at a rate of 0.7ml/min.

For a test of in vivo stability simulation, a polymeric microsphere (Preparation No. 8) in a tube was introduced in a desicator having water at the bottom thereof, with a humidity of 100%, and then was put in a 40°C stability tester. In a predetermined interval (4 weeks, 2 months), a sample (n=3) was collected, dissolved in acetonitrile, and diluted with methanol. Then, the change of a drug content and a related compound was analyzed.

As a result, as noted in Table 24, the polymeric microsphere of the present invention was found to have a related compound of an average value of 0.17%. In general, a related compound is regulated in such a manner that a total of related compounds are 1% or less with respect to a drug raw material, and an individual related compound is 0.1% or less. Thus, it was determined that in the inventive polymeric microsphere preparation process, the stability was achieved.

[Table 24]

| Analysis result of a related compound in a polymeric microsphere | | |
|---|---|---|
| Preparation no | Total of related compounds (%) | Average of total of related compounds (%) |
| 8 | 0.18 | 0.17 |
| | 0.15 | |
| | 0.17 | |

Also, through GPC analysis, the molecular weight of PLGA before/after the preparation was measured. As a result, as noted in Table 25, it was found that most polymers show no change in their molecular weights.

[Table 25]

| Measurement result of a molecular weight of a polymer used in the preparation | | |
|---|---|---|
| Preparation no. | Molecular weight(Mw) | Molecular weight preservation (%) |
| 1 | 38,000 | 65.5 |
| 2 | 24,412 | 84.2 |
| 3 | 79,870 | 68.3 |
| 4 | 37,026 | 94.9 |
| 5 | 69,843 | 105.7 |
| 6 | 23,937 | 108.3 |
| 7 | 27,737 | 95.6 |
| 8 | 73,065 | 110.5 |

As a result of in vivo stability simulation, as noted in Table 26, the average content (%) of a total of related compounds in respective periods during the entire period was 0.23 or less, and a change of a drug content was less than 10%. Thus, it was determined that the inventive preparation method shows a high stability.

[Table 26]

| Result of in vivo stability simulation | | | |
|---|---|---|---|
| Period | Total of related compounds (%) | Drug content % | relative content % |
| Start | 0.17 | 33.341.19 | 100.0 |
| 4weeks | 0.12 | 33.951.31 | 101.8 |
| 2 months | 0.23 | 30.220.14 | 90.6 |

**<10-4> Test 1 on persistence of a prepared anastrozole-containing** polymeric microsphere - **in vitro release test**

In order to determine the persistence of drug release of a polymeric microsphere prepared by the inventive method, in vitro drug release test was carried out.

In a dialysis membrane (MWCO = 12 to 14KD), a polymeric microsphere was introduced. Then, the dialysis membrane was filled with PBS, and was placed in PBS with temperature equilibrium, followed by continuous stirring at a 37 °C thermostat. Then, in a predetermined time interval, the amount of released drug was measured. When the drug release is completed, the remaining polymeric microsphere was vacuum-dried, and then, the amount of remaining drug was measured.

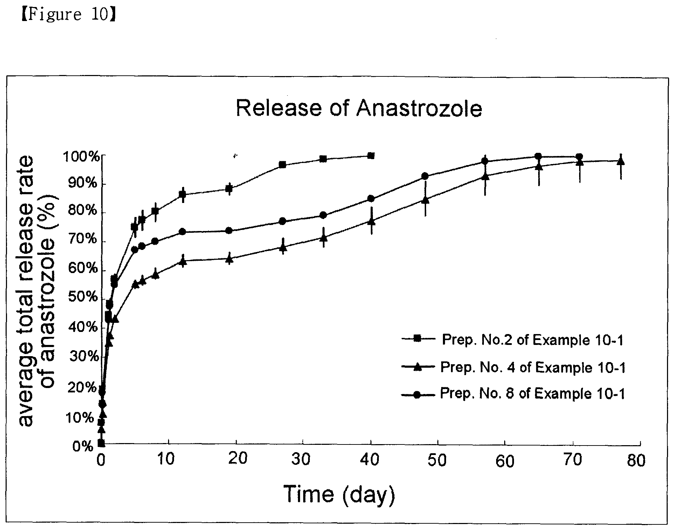

As a result, as shown in FIG. 10, it was determined that after the drug release, polymeric microspheres of all formulations hardly have remaining drugs. Also, according to the kinds of the polymer compounds used in the preparation, as the ratio of lactide was increased, the drug was slowly released. Meanwhile, it was determined that as a drug content was increased, the release rate was increased. Thus, it was determined that it is possible to adjust the release rate by adjusting the kind of the polymer compound used in the polymeric microsphere preparation, and the amount of the loaded drug.

**<10-5> Test 2 on persistence of a prepared anastrozole-containing** polymeric microsphere - **animal test**

In order to determine injectability and dispersibility, a prepared polymeric microsphere was added with a saline solution containing carboxymethyl cellulose (CMC) and tween 20, so as to provide a suspension. The suspension was loaded into a syringe (19G or 20G) and then injected in an amount of 1ml so as to determine the injectability. The result was determined at a concentration of 10% of polymeric microsphere /suspension (w/v).

When a suspension was administered to a rat through injection, each preparation was suspended in an excipient solution (saline including CMC and tween 20) before injection. Female SD rats (aged 9 weeks) were used, and the suspension was administered in an amount of 20mg/kg through intramuscular injection. Then, at a predetermined time interval, the blood was collected, and the concentration of anastrozole in the blood was measured.

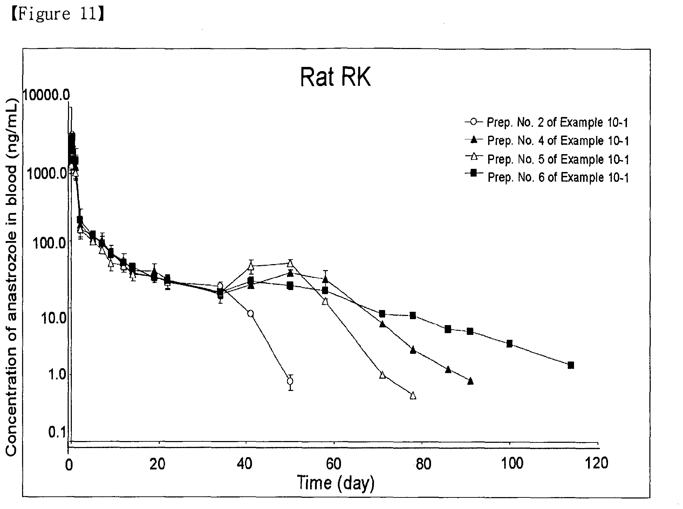

As a result, as shown in FIG. 11, the polymeric microsphere prepared by the inventive method was found to release a drug for a maximum of 120 days.

[Industrial Applicability]

As described above, the present invention provides a novel a method for preparing microsphere and microspheres produced by thereby. More particularly, the present invention relates to a method for preparing a polymeric microsphere comprising the steps of: mixing a water-insoluble organic solvent with a dispersion solvent; mixing a polymer compound, a drug and a water-insoluble organic solvent so as to prepare a dispersed phase; mixing the dispersed phase with the dispersion solvent mixed with the water-insoluble organic solvent so as to prepare an emulsion; and adding a base or an acid to the prepared emulsion so as to remove the water-insoluble organic solvent from the emulsion and a polymeric microsphere thereby, and a composition for drug delivery comprising the microspheres. In the inventive preparation method, it is possible to conveniently prepare a drug-containing polymeric microsphere within a short time using a small amount of water, with the generation of a minimized amount of waste solution without a requirement of a conventional solvent-evaporation or solvent-extraction process. Also, since a low concentration of remaining solvent within a prepared polymeric microsphere can be maintained, it is effective in preparation in sustained-release type medicines.

**Claims**

1. A method for preparing a polymeric microsphere, comprising the steps of:

   (a) mixing a water-insoluble organic solvent with a dispersion solvent;
   (b) mixing a polymer compound, a drug and a water-insoluble organic solvent so as to prepare a dispersed phase;
   (c) mixing the dispersed phase of step (b) with the dispersion solvent mixed with the water-insoluble organic solvent of step (a) so as to prepare an O/W (oil-in-water), O/O (oil-in-oil) or W/O/W (water-in oil-in-water) type emulsion;
   (d) adding a base or an acid to the emulsion of step (c) so as to remove the water-insoluble organic solvent from the emulsion and
   (e) obtaining the polymeric microsphere prepared of step (d) and re-dispersing the obtained polymeric microsphere in a warmed dispersion solvent,

   wherein the water-insoluble organic solvent in the steps (a) and (b) is selected from the group consisting of methyl acetate, ethyl acetate, propyl acetate, isopropyl acetate, butyl acetate, methyl formate, ethyl formate, isopropyl formate, propyl formate, butyl formate, methyl dichloroacetate, methyl chloroacetate, ethyl chloroacetate, ethyl dichloroacetate, methyl fluoroacetate, methyl difluoroacetate, ethyl fluoroacetate, ethyl difluoroacetate, maleic anhydride, acetic anhydride, propionic anhydride, phosphoric anhydride, acetamide, propionamide, and butylamide,
   wherein the dispersion solvent is polyvinyl alcohol solution, or aqueous solution of polysorbates or a co-solvent thereof, or a non-aqueous dispersion solvent which is selected from the group consisting of silicone oil containing glycerin esters of fatty acids or lecithin, vegetable oil, toluene, and xylene,
   wherein the base is selected from the group consisting of sodium hydroxide (NaOH), lithium hydroxide (LiOH), potassium hydroxide (KOH), ammonium hydroxide ($NH_4OH$), copper hydroxide ($Cu(OH)_2$), and iron hydroxide ($Fe(OH)_3$),
   wherein, in the step (c), a volumetric ratio of the dispersed phase of step (b) and the dispersion solvent of step (a) which is mixed with water-insoluble organic solvent is 1:4 to 20, and
   wherein the temperature of the warmed dispersion solvent in the step (e) is 30°C to 50°C.

2. The method of claim 1, wherein the water-insoluble organic solvent of step (b) is identical to the water-insoluble organic solvent of step (a).

3. The method of claim 1, wherein the temperature of the emulsion of the step (c) and the step (d) is 0 °C to 35°C.

4. The method of claim 1, wherein the polymer compound is selected from the group consisting polylactic acid, polylactide, polylactic-co-glycolic acid, polylactide-co-glycolide (PLGA), polyphosphazene, polyiminocarbonate, polyphosphoester, polyanhydride, polyorthoester, lactic acid-caprolactone copolymer, polycaprolactone, polyhydroxyvalerate, polyhydroxybutyrate, polyamino acid, lactic acid-amino acid copolymer, and a mixture thereof.

5. The method of claim 1, wherein the polymer compound is polylactide-co-glycolide (PLGA).

6. The method of claim 1, wherein the drug is/are one or more selected from the group consisting of progesterone, haloperidol, thiothixene, olanzapine, clozapine, bromperidol, pimozide, risperidone, ziprasidone, diazepma, ethyl loflazepate, alprazolam, nemonapride, fluoxetine, sertraline, venlafaxine, donepezil, tacrine, galantamine, rivastigmine, selegiline, ropinirole, pergolide, trihexyphenidyl, bromocriptine, benztropine, colchicine, nordazepam, etizolam, bromazepam, clotiazepam, mexazolum, buspirone, goserelin acetate, somatotropin, leuprolide acetate, octreotide, cetrorelix, sandostatin acetate, gonadotropin, fluconazole, itraconazole, mizoribine, cyclosporin, tacrolimus, naloxone, naltrexone, cladribine, chlorambucil, tretinoin, carmusitne, anagrelide, doxorubicin, anastrozole, idarubicin, cisplatin, dactinomycin, docetaxel, paclitaxel, raltitrexed, epirubicin, letrozole, mefloquine, primaquine, oxybutynin, tolterodine, allylestrenol, lovostatin, simvastatin, provastatin, atrovastatin, alendronate, salcatonin, raloxifene, oxadrolone, conjugated estrogen, estradiol, estradiol valerate, estradiol benzoate, ethinyl estradiol, etonogestrel, levonorgestrel, tibolone, piroxicam and norethisterone.

7. The method of claim 1, wherein the acid is selected from the group consisting of hydrochloric acid (HCl), nitric acid ($HNO_3$), sulfuric acid ($H_2SO_4$), acetic acid ($CH_3COOH$), boric acid ($H_3BO_3$) and carbonic acid ($H_2CO_3$).

**Patentansprüche**

1. Verfahren zur Herstellung einer polymeren Mikrosphäre, umfassend die folgenden Schritte:

(a) Mischen eines wasserunlöslichen organischen Lösungsmittels mit einem Dispersionslösungsmittel;
(b) Mischen einer Polymerverbindung, eines Medikaments und eines wasserunlöslichen organischen Lösungsmittels, um so eine dispergierte Phase herzustellen;
(c) Mischen der dispergierten Phase von Schritt (b) mit dem mit dem wasserunlöslichen organischen Lösungsmittel von Schritt (a) gemischten Dispersionslösungsmittel, um so eine Emulsion vom Typ O/W (Öl-in-Wasser), O/O (Öl-in-Öl) oder W/O/W (Wasser-in-Öl-in-Wasser) herzustellen;
(d) Zugeben einer Base oder einer Säure zu der Emulsion von Schritt (c), um so das wasserunlösliche organische Lösungsmittel aus der Emulsion zu entfernen, und
(e) Erhalten der hergestellten polymeren Mikrosphäre von Schritt (d) und Redispergieren der erhaltenen polymeren Mikrosphäre in einem erwärmten Dispersionslösungsmittel,

wobei das wasserunlösliche organische Lösungsmittel in den Schritten (a) und (b) ausgewählt ist aus der Gruppe bestehend aus Methylacetat, Ethylacetat, Propylacetat, Isopropylacetat, Butylacetat, Methylformiat, Ethylformiat, Isopropylformiat, Propylformiat, Butylformiat, Methyldichlorlacetat, Methylchlorlacetat, Ethylchlorlacetat, Ethyldifluoracetat, Methylfluoracetat, Methyldifluoracetat, Ethylfluoracetat, Ethyldifluoracetat, Maleinsäureanhydrid, Essigsäureanhydrid, Propionsäureanhydrid, Phosphorsäureanhydrid, Acetamid, Propionamid und Butylamid,
wobei das Dispersionslösungsmittel eine Polyvinylalkohollösung oder eine wässrige Lösung von Polysorbaten oder ein Co-Lösungsmittel davon ist, oder ein nichtwässriges Dispersionslösungsmittel ist, das ausgewählt ist aus der Gruppe bestehend aus Silicon-Öl, das Glycerinester von Fettsäuren oder Lecithin enthält, pflanzlichem Öl, Toluol und Xylol,
wobei die Base ausgewählt ist aus der Gruppe bestehend aus Natriumhydroxid (NaOH), Lithiumhydroxid (LiOH), Kaliumhydroxid (KOH), Ammoniumhydroxid ($NH_4OH$), Kupferhydroxid ($Cu(OH)_2$) und Eisenhydroxid ($Fe(OH)_3$),
wobei in Schritt (c) ein Volumenverhältnis der dispergierten Phase von Schritt (b) und des Dispersionslösungsmittels von Schritt (a), das mit dem wasserunlöslichen organischen Lösungsmittel gemischt wird, 1:4 bis 20 ist, und
wobei die Temperatur des erwärmten Dispersionslösungsmittels in Schritt (e) 30 °C bis 50 °C beträgt.

2. Verfahren nach Anspruch 1, wobei das wasserunlösliche organische Lösungsmittel von Schritt (b) identisch mit dem wasserunlöslichen organischen Lösungsmittel von Schritt (a) ist.

3. Verfahren nach Anspruch 1, wobei die Temperatur der Emulsion von Schritt (c) und Schritt (d) 0 °C bis 35 °C beträgt.

4. Verfahren nach Anspruch 1, wobei die Polymerverbindung ausgewählt ist aus der Gruppe bestehend aus Polymilchsäure, Polylactid, Polymilch-co-Glycolsäure, Polylactid-co-Glycolid (PLGA), Polyphosphazen, Polyiminocarbonat, Polyphosphoester, Polyanhydrid, Polyorthoester, Milchsäure-Caprolacton-Copolymer, Polycaprolacton, Polyhydroxyvalerat, Polyhydroxybutyrat, Polyaminosäure, Milchsäure-Aminosäure-Copolymer und einer Mischung davon.

5. Verfahren nach Anspruch 1, wobei die Polymerverbindung Polylactid-co-Glycolid (PLGA) ist.

6. Verfahren nach Anspruch 1, wobei das Medikament eines oder mehrere der Substanzen ist, ausgewählt aus der Gruppe bestehend aus Progesteron, Haloperidol, Thiothixen, Olanzapin, Clozapin, Bromperidol, Pimozid, Risperidon, Ziprasidon, Diazepma, Ethylloflazepat, Alprazolam, Nemonaprid, Fluoxetin, Sertralin, Venlafaxin, Donepezil, Tacrin, Galantamin, Rivastigmin, Selegilin, Ropinirol, Pergolid, Trihexyphenidyl, Bromocriptin, Benztropin, Colchicin, Nordazepam, Etizolam, Bromazepam, Clotiazepam, Mexazolum, Buspiron, Goserelinacetat, Somatotropin, Leuprolideacetat, Octreotid, Cetrorelix, Sandostatinacetat, Gonadotropin, Fluconazol, Itraconazol, Mizoribin, Cyclosporin, Tacrolimus, Naloxon, Naltrexon, Cladribin, Chlorambucil, Tretinoin, Carmusitn, Anagrelid, Doxorubicin, Anastrozol, Idarubicin, Cisplatin, Dactinomycin, Docetaxel, Paclitaxel, Raltitrexed, Epirubicin, Letrozol, Mefloquin, Primaquin, Oxybutynin, Tolterodin, Allylestrenol, Lovostatin, Simvastatin, Provastatin, Atrovastatin, Alendronat, Salcatonin, Raloxifen, Oxadrolon, konjugiertes Östrogen, Estradiol, Estradiol Valerat, Estradiolbenzoat, Ethinylestradiol, Etonogestrel, Levonorgestrel, Tibolon, Piroxicam und Norethisteron.

7. Verfahren nach Anspruch 1, wobei die Säure ausgewählt ist aus der Gruppe bestehend aus Salzsäure (HCl), Salpetersäure ($HNO_3$), Schwefelsäure ($H_2SO_4$), Essigsäure ($CH_3COOH$), Borsäure ($H_3BO_3$) und Kohlensäure ($H_2CO_3$).

**Revendications**

1.  Procédé de préparation d'une microsphère polymère, comprenant les étapes de :

    (a) mélange d'un solvant organique insoluble dans l'eau avec un solvant de dispersion ;
    (b) mélange d'un composé polymère, d'un médicament et d'un solvant organique insoluble dans l'eau de manière à préparer une phase dispersée ;
    (c) mélange de la phase dispersée de l'étape (b) avec le solvant de dispersion mélangé avec le solvant organique insoluble dans l'eau de l'étape (a) de manière à préparer une émulsion de type H/E (huile-dans-eau), H/H (huile-dans-huile) ou E/H/E (eau-dans huile-dans-eau) ;
    (d) addition d'une base ou d'un acide à l'émulsion de l'étape (c) de manière à éliminer le solvant organique insoluble dans l'eau de l'émulsion et
    (e) obtention de la microsphère polymère préparée de l'étape (d) et la redispersion de la microsphère polymère obtenue dans un solvant de dispersion chauffé,

    dans lequel le solvant organique insoluble dans l'eau dans les étapes (a) et (b) est choisi dans le groupe constitué par l'acétate de méthyle, l'acétate d'éthyle, l'acétate de propyle, l'acétate d'isopropyle, l'acétate de butyle, le formiate de méthyle, le formiate d'éthyle, le formiate d'isopropyle, le formiate de propyle, le formiate de butyle, le dichloroacétate de méthyle, le chloroacétate de méthyle, le chloroacétate d'éthyle, le dichloroacétate d'éthyle, le fluoroacétate de méthyle, le difluoroacétate de méthyle, le fluoroacétate d'éthyle, le difluoroacétate d'éthyle, l'anhydride maléique, l'anhydride acétique, l'anhydride
    propionique, l'anhydride phosphorique, l'acétamide, le propionamide et le butylamide,
    dans lequel le solvant de dispersion est une solution d'alcool polyvinylique, ou une solution aqueuse de polysorbates ou d'un de ses co-solvants ou d'un solvant de dispersion non aqueux qui est choisi dans le groupe constitué par de l'huile de silicone contenant des esters d'acides gras de glycérine ou de la lécithine, de l'huile végétale, du toluène et du xylène,
    dans lequel la base est choisie dans le groupe constitué par l'hydroxyde de sodium (NaOH), l'hydroxyde de lithium (LiOH), l'hydroxyde de potassium (KOH), l'hydroxyde d'ammonium ($NH_4OH$), l'hydroxyde de cuivre ($Cu(OH)_2$) et l'hydroxyde de fer ($Fe(OH)_3$),
    dans lequel, dans l'étape (c), un rapport volumétrique de la phase dispersée de l'étape (b) et du solvant de dispersion de l'étape (a) qui est mélangé avec le solvant organique insoluble dans l'eau est de 1 : 4 à 20, et
    dans lequel la température du solvant de dispersion chauffé dans l'étape (e) est de 30 à 50 °C.

2.  Procédé selon la revendication 1, dans lequel le solvant organique insoluble dans l'eau de l'étape (b) est identique au solvant organique insoluble dans l'eau de l'étape (a).

3.  Procédé selon la revendication 1, dans lequel la température de l'émulsion de l'étape (c) et de l'étape (d) est de 0 à 35 °C.

4.  Procédé selon la revendication 1, dans lequel le composé polymère est choisi dans le groupe constitué par l'acide polylactique, le polylactide, l'acide polylactique-co-glycolique, le polylactide-co-glycolide (PLGA), le polyphosphazène, le polyiminocarbonate, le polyphosphoester, le polyanhydride, le polyorthoester, le copolymère acide lactique-caprolactone, la polycaprolactone, le polyhydroxyvalérate, le polyhydroxybutyrate, l'acide polyamino, le copolymère acide lactique-acide aminé et un de leurs mélanges.

5.  Procédé selon la revendication 1, dans lequel le composé polymère est le polylactide-co-glycolide (PLGA).

6.  Procédé selon la revendication 1, dans lequel le médicament est/sont un ou plusieurs choisis dans le groupe constitué par la progestérone, l'halopéridol, le thiothixène, l'olanzapine, la clozapine, le brompéridol, le pimozide, la rispéridone, la ziprasidone, le diazépma, le loflazépate d'éthyle, l'alprazolam, le némonapride, la fluoxétine, la sertraline, la venlafaxine, le donépézil, la tacrine, la galantamine, la rivastigmine, la sélégiline, le ropinirole, le pergolide, le trihexyphénidyle, la bromocriptine, la benztropine, la colchicine, le nordazépam, l'étizolam, le bromazépam, le clotiazépam, le mexazolum, la buspirone, la l'aéctate de goséréline, la somatotropine, l'acétate de leuprolide, l'octréotide, le cétrorélix, l'acétate de sandostatine, la gonadotropine, le fluconazole, l'itraconazole, la mizoribine, la cyclosporine, le tacrolimus, la naloxone, la naltrexone, la cladribine, le chlorambucil, la trétinoïne, la carmustine, l'anagrélide, la doxorubicine, l'anastrozole, l'idarubicine, le cisplatine, la dactinomycine, le docétaxel, le paclitaxel, le raltitrexed, l'épirubicine, le létrozole, la méfloquine, la primaquine, l'oxybutynine, la toltérodine, l'allylestrénol, la lovostatine, la simvastatine, la provastatine, l'atrovastatine, l'alendronate, la salcatonine, le raloxifène, l'oxadrolone, l'oestrogène conjugué, l'estradiol, le valérate d'estradiol, le benzoate d'estradiol, l'estradiol d'éthinyle, l'étonogestrel, le lévonorgestrel, la tibolone, le piroxicam et la noréthistérone.

7. Procédé selon la revendication 1, dans lequel l'acide est choisi dans le groupe constitué par l'acide chlorhydrique (HCl), l'acide nitrique ($HNO_3$), l'acide sulfurique ($H_2SO_4$), l'acide acétique ($CH_3COOH$), l'acide borique ($H_2BO_3$) et l'acide carbonique ($H_2CO_3$).

[Figure 1]

【Figure 2】

【Figure 3】

[Figure 4]

【Figure 5】

Fig. 6

【Figure 7】

【Figure 8】

【Figure 9】

【Figure 10】

Release of Anastrozole

- Prep. No.2 of Example 10-1
- Prep. No. 4 of Example 10-1
- Prep. No. 8 of Example 10-1

【Figure 11】

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6884435 B
- US 3691090 A
- US 3891570 A
- US 6270700 B
- US 6572894 B
- US 4389840 A
- US 4530840 A
- US 6544559 B
- US 6368632 B
- US 6403114 B
- KR 100918092